(19) Europäisches Patentamt
European Patent Office
Office européen des brevets

(11) Veröffentlichungsnummer: **0 005 682**
**B1**

(12) # EUROPÄISCHE PATENTSCHRIFT

(45) Veröffentlichungstag der Patentschrift: **25.01.84**

(51) Int. Cl.³: **C 07 H 23/00,** A 61 K 31/70

(21) Anmeldenummer: **79810040.0**

(22) Anmeldetag: **18.05.79**

(54) **Silyl-glucosaminderivate, Verfahren zu deren Herstellung und diese enthaltende Arzneimittel.**

(30) Priorität: **18.05.78 CH 5394/78**

(43) Veröffentlichungstag der Anmeldung: **28.11.79 Patentblatt 79/24**

(45) Bekanntmachung des Hinweises auf die Patenterteilung: **25.01.84 Patentblatt 84/4**

(84) Benannte Vertragsstaaten: **AT BE CH DE FR GB IT LU NL SE**

(56) Entgegenhaltungen:
**DE - A - 2 450 355**
**FR - A - 2 089 326**
**FR - A - 2 319 373**
**FR - A - 2 343 484**
**FR - A - 2 355 505**
**FR - A - 2 358 159**

(73) Patentinhaber: **CIBA-GEIGY AG, Patentabteilung Postfach, CH-4002 Basel (CH)**

(72) Erfinder: **Baschang, Gerhard, Dr., Bückenweg 7, CH-4126 Bettingen (CH)**
Erfinder: **Dietrich, Felix M., Prof. Dr., Marignanostrasse 64, CH-4059 Basel (CH)**
Erfinder: **Gisler, Roland, Dr., Ob d. Hügliacker 15, CH-4102 Binningen (CH)**
Erfinder: **Hartmann, Albert, Dr., Steingasse 21A, D-7889 Grenzach (DE)**
Erfinder: **Stanek, Jaroslav, Dr., Florastrasse 6, CH-4127 Birsfelden (CH)**
Erfinder: **Tarcsay, Lajos, Dr., Muttenzerstrasse 25, D-7889 Grenzach-Wyhlen (DE)**

(74) Vertreter: **Zumstein, Fritz sen., Dr. et al, Bräuhausstrasse 4, D-8000 München 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# Silyl-Glucosaminderivate, Verfahren zu deren Herstellung und diese enthaltende Arzneimittel

Die Erfindung betrifft neue Silyl-Glucosaminderivate, Verfahren zu deren Herstellung sowie pharmazeutische Präparate, enthaltend diese Verbindungen.

Die Erfindung betrifft insbesondere Silyl-Glucosaminderivate der Formel

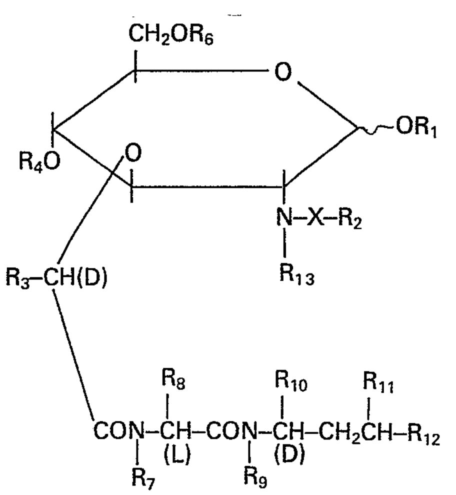

(I)

worin X eine Carbonylgruppe, $R_1$, $R_4$ und $R_6$ Triniederalkylsilyl, $R_2$ unsubstituiertes oder durch Hydroxy, Niederalkoxy, Triniederalkylsilyloxy, Niederalkanoyloxy, Halogen, Mercapto, Niederalkylmercapto, Triniederalkylsilylmercapto, Niederalkanoylmercapto, Carboxy, Niederalkoxycarbonyl und/oder Carbamoyl substituiertes Niederalkyl oder unsubstituiertes oder durch Niederalkyl, Hydroxy, Niederalkoxy, Niederalkanoyloxy, Triniederalkylsilyloxy, Niederalkylendioxy, Halogen, Amino und/oder Trifluoromethyl mono-, di- oder polysubstituiertes Phenyl, $R_3$, $R_7$ und $R_{13}$ Wasserstoff oder Niederalkyl, $R_8$ Wasserstoff, unsubstituiertes oder durch Hydroxy, Niederalkoxy, Triniederalkylsilyloxy, Niederalkanoyloxy, Halogen, Mercapto, Niederalkylmercapto, Triniederalkylsilylmercapto oder Niederalkanoylmercapto substituiertes Niederalkyl, Cycloalkyl mit 5 oder 6 Kohlenstoffatomen, Cycloalkyl-niederalkyl, dessen Cycloalkylrest 5 oder 6 Kohlenstoffatome enthält, einen unsubstituierten oder im Phenylteil durch Niederalkyl, Hydroxy, Niederalkoxy, Niederalkanoyloxy, Triniederalkylsilyloxy, Niederalkylendioxy, Halogen und/oder Trifluormethyl mono-, di- oder polysubstituierten Phenyl- oder Phenylniederalkylrest, Heterocyclyl oder Heterocyclylniederalkyl mit jeweils 5 oder 6 Ringgliedern und ein oder zwei Stickstoffatomen im gegebenenfalls benzoannellierten heterocyclischen Ring, $R_7$ und $R_8$ zusammen auch Alkylen mit 3 oder 4 Kohlenstoffatomen, $R_9$ Wasserstoff oder Niederalkyl und die Reste $R_{10}$, $R_{11}$ und $R_{12}$ unabhängig voneinander Carboxy, Niederalkoxycarbonyl, Triniederalkylsilyloxycarbonyl oder unsubstituiertes oder durch Alkyl mit 1–18 C-Atomen oder einen unsubstituierten oder im Phenylteil durch Niederalkyl, Hydroxy, Niederalkoxy, Niederalkanoyloxy, Triniederalkylsilyloxy, Niederalkylendioxy, Halogen und/oder Trifluormethyl, mono-, di- oder polysubstituierten Phenyl- oder Phenylniederalkylrest mono- oder disubstituiertes Carbamoyl oder durch Niederalkylen substituiertes Carbamoyl, $R_{10}$ auch durch Carbamoylmethyl oder L-1- oder D-1-Carbamoyläthyl substituiertes Carbamoyl und $R_{11}$ auch Wasserstoff bedeuten.

Alkyl mit bis zu 18 Kohlenstoffatomen in den Resten $R_{10}$, $R_{11}$ und $R_{12}$ ist geradkettiges oder verzweigtes, in beliebiger Stellung gebundenes Alkyl, in erster Linie jedoch Niederalkyl.

Ein obengenannter substituierter Niederalkylrest kann einen, zwei oder mehrere gleiche oder verschiedene Substituenten tragen.

Stickstoffhaltiges Heterocyclyl kann ungesättigt oder auch gesättigt sein und einen ankondensierten Phenylrest (benzoannelliertes Heterocyclyl) enthalten. Genannt seien z. B. der Pyrrol-, Pyridyl- oder Imidazolring.

Cycloalkyl ist besonders Cyclopentyl oder Cyclohexyl.

Die im Zusammenhang mit der vorliegenden Beschreibung und den Patentansprüchen mit «Nieder» bezeichneten Reste und Verbindungen enthalten bis und mit 7 und in erster Linie bis und mit 4 Kohlenstoffatome.

Vorstehend, wie nachfolgend können die Allgemeinbegriffe folgende Bedeutung haben:

Niederalkyl, auch in zusammengesetzten Begriffen, wie Triniederalkylsilyloxy, Cycloalkyl-niederalkyl, Niederalkanoylmercapto usw., kann geradkettig oder verzweigt sowie in beliebiger Stellung gebunden sein, und ist z. B. n-Propyl, n-Butyl, Isobutyl, sek.-Butyl oder tert.-Butyl, ferner n-Pentyl, n-Hexyl, Isohexyl oder n-Heptyl und in erster Linie Methyl oder Äthyl. In Phenyl-, Cycloalkyl- oder Heterocyclylniederalkyl ist der Niederalkylrest insbesondere Methyl oder Äthyl, wobei der Phenyl-, Cycloalkyl- oder Heterocyclylrest die obengenannte Bedeutung besitzt.

Niederalkoxy ist z. B. n-Propoxy, n-Butoxy, Isobutoxy, sek.-Butoxy oder tert.-Butoxy und in erster Linie Methoxy oder Äthoxy.

Niederalkylmercapto ist z. B. n-Propyl-, n-Butyl-, Isobutyl, sek.-Butyl oder tert.-Butylmercapto und in erster Linie Methylmercapto oder Äthylmercapto.

Niederalkylendioxy ist insbesondere Methylendioxy, Äthylen- oder Propylendioxy.

Halogen steht für Fluor oder Brom, vorzugsweise jedoch für Chlor.

Niederalkanoyl ist insbesondere Propionyl oder Butyryl, in erster Linie jedoch Acetyl.

Die neuen Verbindungen der vorliegenden Erfindung können in Form von Gemischen von Isomeren oder von reinen Isomeren vorliegen.

Silylierte Muramylpeptide waren zum Zeitpunkt der vorliegenden Erfindung im Gegensatz zu den korrespondierenden unsilylierten Muramylpeptiden und/oder Verfahren zu deren Herstellung [siehe z. B. FR-A 2343484 (ANVAR), FR-A 2355505 (ANVAR), FR-A 2358159 (ANVAR), DE-A 2450355

(ANVAR) und DE-A 2655500 (CIBA-GEIGY)] nicht bekannt.

In FR-A 2089326, S. 1, Zeilen 6–15, heisst es, man habe gefunden, dass die Silylierung gewisser Produkte mit biologischer Aktivität, wie die Antibiotika und die Blutantikoagulantien, es gestatte, die Geschwindigkeit ihrer Ausscheidung (aus dem Organismus) herabzusetzen, was es ermögliche, die Wirkungsdauer des biologisch aktiven Produktes zu verlängern, ohne dessen gewünschte Wirksamkeit zu verändern. Jedoch sind dieser Patentschrift weder Referenzen noch die Ergebnisse eigener Arbeiten zu entnehmen, die diese sehr allgemein gehaltene Behauptung in ihrer vollen Breite unterstützen könnten. Da die erfindungsgemässen Silylmuramylpeptide lipophil, die entsprechenden unsilylierten Muramylpeptide dagegen hydrophil sind, erwartete der Fachmann am Prioritätszeitpunkt, dass die erfindungsgemässen Verbindungen die Lipidbarriere eines Organismus durchdringen und an einen anderen Wirkungsort gelangen würden als die entsprechenden unsilylierten Verbindungen. Ausserdem war unbekannt, ob die silylierten Muramylpeptide am Wirkungsort zu den freien Verbindungen gespalten werden oder nicht. Es war daher überraschend und unvorhersehbar, dass die silylierten und daher lipophilen Verbindungen der Formel I ebenso wie die entsprechenden unsilylierten hydrophilen Muramylpeptide immunologische Aktivität aufweisen. Damit wird der Stand der Technik mit neuen immunologisch aktiven Verbindungen mit veränderten Eigenschaften bereichert.

Mit den nachstehend beschriebenen Versuchsanordnungen kann gezeigt werden, dass die Verbindungen der vorliegenden Erfindung wertvolle pharmakologische Eigenschaften, insbesondere eine ausgeprägte immunpotenzierende Wirkung, aufweisen:

1. Potenzierung der zellulären Immunität in vivo: Steigerung der Spättyp-Überempfindlichkeit gegen Ovalbumin beim Meerschweinchen

Pirbright Meerschweinchen werden am Tag 0 mit 10 mg Ovalbumin in komplettem Freund'schem Adjuvans durch Injektion von je 0,1 ml eines Antigen-Adjuvans-Gemisches in die beiden Hinterpfoten immunisiert. 4 Wochen später werden Hautreaktionen durch intrakutane Injektion von 100 ug Ovalbumin in 0,1 ml gepufferter physiologischer Salzlösung ausgelöst und aufgrund des 24 Stunden danach anhand der Erythemfläche und der Hautdickenzunahme berechneten Reaktionsvolumens quantifiziert. Die nach 24 Stunden (Spättypreaktion) beobachtete antigegenspezifische Zunahme des Reaktionsvolumens gilt als ein Mass für zellvermittelte Immunität. Ovalbumin ist ein zu schwaches Immunogen, um für sich allein oder in einer Wasserölemulsion mit inkomplettem Freund'schem Adjuvans [10 Teile Ovalbuminlösung in 0,9% NaCl gemischt mit 8,5 Teilen Bayol F (Warenzeichen) und 1,5 Teilen Arlacel A (Warenzeichen)] eine Spättypreaktion zu induzieren, sondern muss für eine effektive Immunisierung in komplettem Adjuvans, dem Mykobakterien zugesetzt sind [5 mg abgetötetes und lyophilisiertes M butyricum pro 10 ml Bayol F (Warenzeichen)/Arlacel A (Warenzeichen)], appliziert werden. Zum Nachweis der immunpotenzierenden Wirkung von Prüfsubstanzen können diese nun an Stelle der Mykobakterien in Dosen von 10 bis 100 µg dem Antigen-Ölgemisch beigemengt werden.

Die erfindungsgemässen Glucosaminpeptide sind in der Lage, den Effekt der Mykobakterien in der beschriebenen Versuchsanordnung nachzuahmen und ihn quantitativ zu übertreffen.

Damit wird gezeigt, dass Verbindungen der beschriebenen Art zelluläre Immunität erheblich zu steigern vermögen, und zwar sowohl in Mischung mit dem Antigen selber (Adjuvanseffekt im engeren Sinne), als auch bei zeitlich und örtlich von der Antigeninjektion getrennter Zufuhr (systemische Immunpotenzierung).

2. Potenzierung der humoralen Immunität in vivo: Steigerung der Antikörperproduktion gegen bovines Serumalbumin (BSA) bei der Maus

NMRI Mäuse werden durch intraperitoneale (i.p.) Injektion von 10 µg präzipitatfreiem BSA am Tag 0 immunisiert. 9, 15 und 29 Tage später werden Serumproben entnommen und auf ihren Gehalt an anti-BSA-Antikörpern mit einer passiven Haemagglutinationstechnik untersucht. In der verwendeten Dosis ist lösliches BSA für die Empfängertiere subimmunogen, d. h. es vermag keine oder nur eine ganz geringfügige Produktion von Antikörpern auszulösen. Zusätzliche Behandlung der Mäuse mit gewissen immunpotenzierenden Stoffen vor oder nach der Antigengabe führt zu einem Anstieg der Antikörpertiter im Serum. Der Effekt der Behandlung wird durch den erreichten Scorewert, d. h. durch die Summe der $Log_2$ Titerdifferenzen an den drei Blutungstagen ausgedrückt.

Verbindungen der vorliegenden Erfindung sind in der Lage, bei intraperitonealer oder subkutaner (s.c.) Applikation von 100–300 mg/kg/Tier an fünf aufeinanderfolgenden Tagen (Tag 0 bis 4) nach Immunisierung mit BSA die Antikörperproduktion gegen BSA signifikant zu steigern.

Der immunstimulierende Effekt der genannten Verbindungen ist im Gegensatz zu dem anderer bakterieller Immunoleptica (z. B. LPS aus E. coli) antigenabhängig: Injektion der neuen Verbindungen hat nur in BSA-immunisierten, nicht aber in nicht-immunisierten Mäusen eine Erhöhung der anti-BSA-Titer zur Folge. Bemerkenswerterweise ist die s.c. Gabe der genannten Verbindungen ebenso effektiv wie die i.p. Applikation, d. h. die beobachtete immunpotenzierende Wirkung ist systemisch und hängt nicht davon ab, dass das Stimulans über die gleiche Route wie das Antigen, bzw. mit dem Antigen gemischt verabreicht werden muss, wie dies bei klassischen Adjuvantien der Fall ist.

Durch die geschilderten Versuche wird gezeigt, dass Verbindungen der beschriebenen Art auch die humorale Immunität spezifisch zu steigern vermögen, dass sie die immunologische Reizbeantwortung verbessern, und dass ihre immunpotenzierenden Effekte auf einer systemischen Aktivierung des Immunapparates beruhen.

3. Potenzierung der humoralen Immunität in vitro: T-Zell-substituierender Effekt bei der Antikörperantwort von Mäusemilzzellen gegen Schaferythrocyten (SE)

Für die Induktion einer Antikörperantwort werden in vielen Fällen aus dem Thymus stammende Lymphocyten (T-Zellen) benötigt. Diese Zellen kooperieren mit den Vorläufern antikörperbildender Lymphocyten (B-Zellen) und helfen ihnen auf Stimulierung mit sogenannten T-abhängigen Antigenen mit Proliferation, Differenzierung und Antikörpersynthese zu reagieren. Milzzellsuspensionen kongenital athymischer nu/nu Mäuse enthalten keine funktionellen T-Zellen und vermögen z. B. in vitro in Gegenwart von SE keine anti-SE-Antikörper zu bilden. Die Verbindungen der vorliegenden Erfindung sind überraschenderweise in der Lage, T-Zellen in solchen Kulturen funktionell zu ersetzen und eine Antikörperantwort gegen SE zu ermöglichen. Zusatz dieser Stoffe zu nu/nu Milzzellkulturen in Gegenwart von SE führt innert 4 Tagen zu einem erheblichen Anstieg der Zahl antikörperbildender Zellen. Die Befunde zeigen, dass die genannten Verbindungen die humorale Antikörperbildung in vitro zu steigern und einen Defekt des T-Zell-Systems zu kompensieren vermögen.

4. Selektive Mitogenität für B-Zellen: Proliferationsfördernder Effekt in B-Lymphocyten-Kulturen

Suspensionen hoch angereicherter B-Lymphocyten (Lymphknotenzellen kongenital athymischer nu/nu Mäuse) sowie weitgehend reiner unreifer und reifer T-Lymphocyten (Thymuszellen bzw. cortisonresistente, d. h. 48 Stunden nach einer Cortisoninjektion persistierende Thymuszellen von Balb/c-Mäusen) werden in Gegenwart der Prüfsubstanzen während drei Tagen inkubiert. Die Inkorporation von $H^3$-Thymidin in die Lymphocyten während der letzten 18 Stunden der Kulturperiode gilt als Mass für die Proliferationsaktivität.

Die erfindungsgemässen Verbindungen sind für B-Lymphocyten (d. h. für die Vorläufer der antikörperbildenden Zellen), nicht aber für T-Lymphocyten mitogen.

Sie sind somit in der Lage, die Proliferation von Lymphocyten anzuregen, die an der humoralen Immunantwort beteiligt sind.

5. Verträglichkeit

Obwohl Verbindungen der beschriebenen Art ihre potenzierende Wirkung am Meerschweinchen beispielsweise bereits nach einer Einzeldosis von 0,05 mg/kg s.c., an der Maus nach Applikation von 5 mal 10 mg/kg s.c. entfalten, werden auch bei Applikation von 5 mal 300 mg/kg i.p. an Mäusen keine toxischen Effekte beobachtet. Die genannten Stoffe verfügen deshalb über eine ausgezeichnete therapeutische Breite.

Die erfindungsgemässen Verbindungen haben die Fähigkeit, einerseits bei Mischung mit einem Antigen dessen Immunogenität zu erhöhen, andererseits bei systemischer Applikation die immunologische Reaktivität des behandelten Organismus zu steigern. Dabei sind die genannten Stoffe in der Lage, sowohl die zelluläre wie die humorale Immunität zu fördern und die für die Antikörperbildung verantwortlichen Lymphocyten zu aktivieren.

Die neuen Verbindungen können somit als Adjuvantien in Mischung mit Impfstoffen dazu benützt werden, den Impferfolg zu verbessern und den durch humorale Antikörper und/oder zelluläre Immunität vermittelten Infektionsschutz gegenüber bakteriellen, viralen oder parasitären Erregern zu verbessern.

Schliesslich eignen sich die beschriebenen Verbindungen in Mischung mit verschiedensten Antigenen als Adjuvantien bei der experimentellen und industriellen Herstellung von Antiseren für Therapie und Diagnostik und bei der Induktion von immunologisch aktivierten Lymphocytenpopulationen für Zelltransferverfahren.

Darüber hinaus können die neuen Verbindungen auch ohne gleichzeitige Antigenzufuhr dazu benützt werden, bereits unterschwellig ablaufende Immunreaktionen bei Mensch und Tier zu fördern. Die Verbindungen eignen sich demnach besonders für die Stimulation der körpereigenen Abwehr, z. B. bei chronischen und akuten Infektionen oder bei selektiven (antigenspezifischen) immunologischen Defekten sowie bei angeborenen, aber auch bei erworbenen allgemeinen (d. h. nicht antigenspezifischen) immunologischen Defektzuständen, wie sie im Alter, im Verlauf schwerer Primärerkrankungen und vor allem nach Therapie mit ionisierenden Strahlen oder mit immunosupressiv wirkenden Hormonen auftreten. Die genannten Stoffe können somit vorzugsweise auch in Kombination mit antiinfektiösen Antibiotika, Chemotherapeutika oder anderen Heilverfahren verabreicht werden, um immunologischen Schädigungen entgegenzuwirken. Schliesslich sind die beschriebenen Stoffe auch zur allgemeinen Prophylaxe von Infektionskrankheiten bei Mensch und Tier geeignet.

Die Erfindung betrifft insbesondere Verbindungen der Formel I, worin X den Carbonylrest, $R_1$, $R_4$ und $R_6$ Triniederalkylsilyl, in erster Linie Trimethylsilyl, $R_2$ gegebenenfalls durch Halogen, Hydroxy oder Triniederalkylsilyloxy substituiertes Niederalkyl oder gegebenenfalls durch Niederalkyl, Niederalkoxy, Halogen oder Amino substituiertes Phenyl, $R_3$ Wasserstoff oder Alkyl mit 1–3 Kohlenstoffatomen, $R_7$, $R_9$ und $R_{13}$ Wasserstoff, $R_8$ Niederalkyl, Hydroxyniederalkyl, Triniederalkylsilyloxyniederalkyl, Mercaptoniederalkyl oder Triniederalkylsilylmercaptoniederalkyl, $R_7$ und $R_8$ zusammen auch einen Trimethylenrest, $R_{10}$ und $R_{12}$ unabhängig voneinander Carboxy, Niederalkoxycarbonyl, Triniederalkylsilyloxycarbonyl oder unsubstituiertes oder durch Alkyl mit 1–18 C-Atomen oder einen unsubstituierten oder im Phenylteil durch Niederalkyl, Hydroxy, Niederalkoxy, Niederalkanoyloxy, Triniederalkylsilyloxy, Niederalkylendioxy, Halogen und/oder Trifluormethyl mono-, di- oder polysubstituierten Phenyl- oder Phenylniederalkylrest mono- oder disubstituiertes Carbamoyl oder durch Niederalkylen substituiertes Carbamoyl, $R_{10}$ auch durch Carbamoylmethyl oder L-1- oder D-1-Carbamoyläthyl substituiertes Carbamoyl und $R_{11}$ Wasserstoff, Triniederalkylsilyloxycarbonyl oder einen Carboxyrest bedeuten.

Besonders wertvoll sind auch Verbindungen obiger Formel worin $R_3$ Wasserstoff darstellt.

Hervorzuheben sind ferner Verbindungen obiger Formel I, worin X den Carbonylrest, $R_1$, $R_4$ und $R_6$ Triniederalkylsilyl, in erster Linie Trimethylsilyl, $R_2$ Niederalkyl oder Phenyl, $R_3$ Wasserstoff oder Methyl, $R_7$, $R_9$ und $R_{13}$ Wasserstoff, $R_8$ Niederalkyl, Hydroxyniederalkyl, Triniederalkylsilyloxyniederalkyl, Mercaptomethyl oder Triniederalkyl-silylmercaptomethyl, $R_7$ und $R_8$ zusammen auch den Trimethylenrest, $R_{10}$ und $R_{12}$ unabhängig voneinander Carboxy, Triniederalkylsilyloxycarbonyl oder Carbamoyl und $R_{11}$ Wasserstoff, Triniederalkylsilyloxycarbonyl oder Carboxy bedeuten.

In erster Linie sind Verbindungen der Formel I zu erwähnen, worin X den Carbonylrest, $R_1$ $R_4$ und $R_6$ Trimethylsilyl, $R_2$ Niederalkyl oder Phenyl, $R_3$ Wasserstoff oder Methyl, $R_7$, $R_9$ und $R_{13}$ Wasserstoff, $R_8$ Niederalkyl mit 1–3 Kohlenstoffatomen, Hydroxymethyl, 1-Hydroxy-äthyl, Trimethylsilyloxymethyl, Mercaptomethyl oder Trimethylsilylmercaptomethyl, $R_{10}$ Carbamoyl, $R_{11}$ Wasserstoff und $R_{12}$ Carboxy, Trimethylsilyloxycarbonyl oder Carbamoyl bedeuten.

Insbesondere betrifft die Erfindung die neuen, in den Beispielen beschriebenen Verbindungen.

Diese Verbindungen können erhalten werden, wenn man in an sich bekannter Weise eine Verbindung der Formel

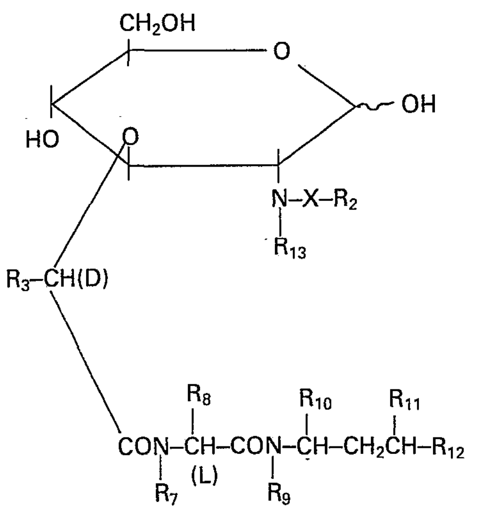

(II)

worin die Substituenten wie oben definiert sind, mit einem einen Triniederalkylsilylrest einführenden Silylierungsmittel umsetzt und erhaltene Silylester der Formel I gegebenenfalls mit Wasser zu den Silyläthern der Formel I aufspaltet.

Als Silylierungsmittel seien insbesondere genannt: Triniederalkyl-silyl-halogenide, besonders -chloride oder -bromide, Hexaniederalkyl-disilazan, Triniederalkylsilylamin, Triniederalkylsilyldiäthylamin, Triniederalkylsilylacetamid, Bis-(triniederalkylsilyl)-acetamide oder Tri-niederalkyl-silylsulfamide, insbesondere die entsprechenden Trimethylsilyl-Verbindungen.

Die Reaktion wird vorzugsweise in einem Lösungsmittel, das keine reaktionsfähigen Hydroxy- oder Aminogruppen enthält, wie Dimethylformamid, Dimethylsulfoxyd oder Acetonitril, Dioxan, Tetrahydrofuran, Dimethoxyäthan oder Chloroform vorgenommen. Man kann zudem in Gegenwart einer wasserfreien Base, wie Triäthylamin, Piperidin, Pyridin oder Imidazol arbeiten. Falls in den Ausgangsstoffen eine freie Carboxyl-, Hydroxy- oder Mercaptogruppe vorhanden ist, können diese unter den Reaktionsbedingungen ebenfalls silyliert werden. Erhaltene Silylester werden aber sehr leicht, z. B. beim Kontakt mit Wasser, gespalten.

Das oben beschriebene Verfahren wird nach an sich bekannten Methoden durchgeführt, in Abwesenheit oder vorzugsweise in Anwesenheit von Verdünnungs- oder Lösungsmitteln, wenn notwendig, unter Kühlen oder Erwärmen, unter erhöhtem Druck und/oder in einer Inertgas-, wie Stickstoffatmosphäre.

Die verwendeten Ausgangsstoffe und ihre Herstellung sind bekannt.

Die Erfindung betrifft auch diejenigen Ausführungsformen des Verfahrens, bei denen man einen Ausgangsstoff unter den Reaktionsbedingungen bildet oder in Form eines reaktionsfähigen Derivats oder Salzes verwendet. Dabei geht man vorzugsweise von solchen Ausgangsstoffen aus, die verfahrensgemäss zu den oben als besonders wertvoll beschriebenen Verbindungen führen.

Die vorliegende Erfindung betrifft ebenfalls pharmazeutische Präparate, welche Verbindungen der Formel I enthalten. Bei den erfindungsgemässen pharmazeutischen Präparaten handelt es sich um solche zur enteralen, insbesondere parenteralen Verabreichung an Warmblüter, welche den pharmakologischen Wirkstoff allein oder zusammen mit einem pharmazeutisch anwendbaren Trägermaterial enthalten. Die Dosierung des Wirkstoffs hängt von der Warmblüter-Spezies, dem Alter und dem individuellen Zustand sowie von der Applikationsweise ab.

Die neuen pharmazeutischen Präparate enthalten von 10% bis 95%, vorzugsweise von 20% bis 90% des Wirkstoffs. Erfindungsgemässe pharmazeutische Präparate können z. B. in Dosiseinheitsform, wie Suppositorien oder Ampullen vorliegen.

Die pharmazeutischen Präparate der vorliegenden Erfindung werden in an sich bekannter Weise, z. B. mittels konventioneller Misch-, Granulier-, Dragier-, Lösungs- oder Lyophilisierungsverfahren hergestellt. Vorzugsweise löst man die neuen Verbindungen in Lecithinen, gegebenenfalls zusammen mit Cholesterin, in einem halogenierten Kohlenwasserstoff, wie Chloroform oder Methylenchlorid, dampft sie dann ein und verteilt die erhaltene Mischung in einer wässrigen Pufferlösung, z. B. phosphatgepuffertes Kochsalz vom pH 7,2, z. B. durch Beschallen.

Man kann sie auch zusammen mit einem geeigneten Trägerstoff zu festen Darreichungsformen verarbeiten. Geeignete Trägerstoffe sind insbesondere Füllstoffe, wie Zucker, z. B. Lactose, Saccharose, Mannit oder Sorbit, Cellulosepräparate und/oder Calciumphosphate, z. B. Tricalciumphosphat oder Calciumhydrogenphosphat, ferner Bindemittel, wie Stärkekleister unter Verwendung z. B. von Mais-, Weizen-, Reis- oder Kartoffelstärke, Gelatine, Traganth, Methylcellulose, Hydroxypropyl-methylcellulose, Natriumcarboxymethylcellulose und/oder Polyvinylpyrrolidon, und/oder, wenn erwünscht Sprengmittel, wie die obgenannten Stärken, ferner Carboxymethylstärke, quervernetztes Polyvinylpyrrolidon, Agar, Alginsäure oder ein Salz davon, wie Natriumalginat, Hilfsmittel sind in erster Linie Fliessregulier- und Schmiermittel, z. B. Kieselsäure, Talk, Stearinsäure oder Salze davon, wie Magnesium- oder Calciumstearate, und/oder Polyäthylenglykol. Dragée-Kerne werden mit geeigneten, gegebenenfalls Magensaft-resistenten Überzügen versehen, wobei man u. a. konzentrierte Zuckerlösungen, welche gegebenenfalls arabischen Gummi, Talk, Polyvinylpyrrolidon, Polyäthylenglycol und/oder Titandioxid enthalten, Lacklösungen in geeigneten organischen Lösungsmitteln oder Lösungsmittelgemischen oder, zur Herstellung von Magensaft-resistenten Überzügen, Lösungen von geeigneten Cellulosepräparaten, wie Acetylcellulosephthalat oder Hydroxypropylmethylcellulosephthalat, verwendet. Den Tabletten oder Dragée-Überzügen können Farbstoffe oder Pigmente, z. B. zur Identifizierung oder zur Kennzeichnung verschiedener Wirkstoffdosen, beigefügt werden.

Die nachfolgenden Beispiele illustrieren die oben beschriebene Erfindung. Temperaturen werden in Celsiusgraden angegeben.

Beispiel 1

0,3 g 2-Benzamido-2-desoxy-3-O-{[L-1-(D-1-carbamoyl-3-carboxypropyl)-carbamoyl-äthyl]-carbamoyl-methyl}-D-glucose löst man in 3 ml Dimethylformamid und versetzt mit 0,4 ml Bis-trimethylsilylacetamid. Nach 5 Stunden bei 35° dampft man im Vakuum zum Sirup ein, aus dem man durch Lösen in absolutem Äther oder absolutem Essigester gebildetes Acetamid abscheiden kann. Nach erneutem Eindampfen erhält man einen farblosen Sirup mit $[\alpha]_D^{20} = +15°$ (c = 0,8, Dioxan) der 2-Benzamido-2-desoxy-3-O-{[L-1-(D-1-carbamoyl-3-trimethylsilyl-carboxy-propyl)-carbamoyl-äthyl]-carbamoyl-methyl}-1,4,6-O-tris-trimethylsilyl-α,β-D-glucose.

Bei Kontakt mit Wasser wird die Trimethylsilylestergruppe rasch verseift, wobei man die 2-Benzamido-2-desoxy-3-O-{[L-1-(D-1-carbamoyl-3-carboxy-propyl)-carbamoyl-äthyl]-carbamoylmethyl}-1,4,6-tris-O-trimethylsilyl-α,β-D-glucose erhält.

Analog erhält man sirupöse 2-Acetamino-2-desoxy-3-O-{[L-1-(D-1-carbamoyl-3-trimethylsilyl-carboxy-propyl)-carbamoyläthyl]-carbamoylmethyl}-1,4,6-tris-O-trimethylsilyl-α,β-D-glucose mit $[\alpha]_D^{20} = -10°$ (c = 0,91, Dioxan).

Beispiel 2

3 g 2-Benzamido-2-desoxy-3-O-{[L-1-(D-1-carbamoyl-3-carboxypropyl)-carbamoyl-äthyl]-carbamoyl-methyl}-D-glucose löst man in 20 ml Dimethylformamid und versetzt mit 4 ml Bis-trimethylsilylacetamid. Nach 5 Stunden bei 35° dampft man im Vakuum zum Sirup ein. Man nimmt in Chloroform auf, schüttelt kurz mit Eiswasser aus und trocknet die organische Phase. Nach erneutem Eindampfen erhält man ein Gemisch des entsprechenden 1,4,6-Tris-trimethylsilyläthertrimethylsilylesters und des zugehörigen Tris-trimethylsilyläthers als freie Carbonsäure.

Beispiel 3

Analog den vorstehenden Beispielen erhält man folgende Trimethylsilyläther-ester, aus denen bei Kontakt mit Wasser die Trimethylsilyläther entstehen:

2-Acetamino-2-desoxy-3-O-{D-1-(L-1-[D-1-carbamoyl-3-trimethylsilyl-carboxy-propyl]-carbamoyl-äthyl)-carbamoyläthyl}-1,4,6-O-tris-trimethylsilyl-α,β-D-glucose, Sirup $[\alpha]_D^{20} = +10°$ (c = 1 Dioxan)

2-Acetamino-2-desoxy-3-O-{D-1-(L-1-[D-1-carbamoyl-3-trimethylsilyl-carboxy-propyl]-carbamoyl-propyl)-carbamoyläthyl}-1,4,6-O-tris-trimethylsilyl-α,β-D-glucose, Sirup.

2-Acetamino-2-desoxy-3-O-{D-1-(L-1-[D-1-carbamoyl-3-trimethylsilyl-carboxy-propyl]-carbamoyl-2-methyl-propyl)-carbamoyläthyl}-1,4,6-O-tris-trimethylsilyl-α,β-D-glucose, Sirup.

2-Propionamino-2-desoxy-3-O-[{L-1-(D-1-carbamoyl-3-trimethylsilyl-carboxy-propyl)-carbamoyl-äthyl}-carbamoyl-methyl]-1,4,6-O-tris-trimethylsilyl-α,β-D-glucose, Sirup.

2-Benzoylamino-2-desoxy-3-O-[{L-1-(D-1-carbamoyl-3-trimethylsilyl-carboxypropyl)-carbamoyl-propyl}-carbamoyl-methyl]-1,4,6-O-tris-trimethylsilyl-α,β-D-glucose, Sirup.

2-Acetamino-2-desoxy-3-O-[{L-1-(D-1-carbamoyl-3-trimethylsilyl-carboxy-propyl)-carbamoyl-propyl}-carbamoylmethyl]-1,4,6-O-tris-trimethylsilyl-α,β-D-glucose, Sirup.

2-Acetamino-2-desoxy-3-O-[{L-1-(D-1-carbamoyl-3-trimethylsilyl-carboxypropyl)-carbamoyl-2-methylpropyl}-carbamoyl-methyl]-1,4,6-O-tris-trimethylsilyl-α,β-D-glucose, Sirup.

2-Acetamino-2-desoxy-3-O-[{L-1-(D-1-N-carbamoylmethyl-carbamoyl-3-trimethylsilyl-carboxy-propyl)-carbamoyl-äthyl}-carbamoylmethyl]-1,4,6-O-tris-trimethylsilyl-α,β-D-glucose, Sirup.

2-Benzoylamino-2-desoxy-3-O-[{L-1-(D-1-N-carbamoylmethyl-carbamoyl-3-trimethylsilyl-carboxypropyl)-carbamoyl-äthyl}-carbamoylmethyl]-1,4,6-O-tris-trimethylsilyl-α,β-D-glucose, Sirup.

2-Pivaloylamino-2-desoxy-3-O-[{L-1-(D-1-carbamoyl-3-trimethylsilyl-carboxypropyl)-carbamoyl-äthyl}-carbamoylmethyl]-1,4,6-tris-O-trimethylsilyl-α,β-D-glucose, Sirup.

2-Propionamino-2-desoxy-3-O-[{L-1-(D-1-trimethylsilyl-carboxy-3-trimethylsilyl-carboxy-propyl)-carbamoyl-äthyl}-carbamoylmethyl]-1,4,6-O-tris-trimethylsilyl-α,β-D-glucose, Sirup.

2-Acetamino-2-desoxy-3-O-[{L-1-(D-1-trimethylsilyl-carboxy-3-trimethylsilyl-carboxy-propyl)-carbamoyl-propyl}-carbamoylmethyl]-1,4,6-O-tris-trimethylsilyl-α,β-D-glucose, Sirup.

2-Acetamino-2-desoxy-3-O-[{L-1-(D-1-carbamoyl-3-trimethylsilyl-carboxy-propyl)-carbamoyl-2-trimethylsilyl-oxy-äthyl}-carbamoylmethyl]-1,4,6-O-tris-trimethylsilyl-α,β-D-glucose, Sirup.

2-Acetamino-2-desoxy-3-O-{D-1-(L-1-[D-1-trimethylsilyl-carboxy-3-trimethylsilyl-carboxy-propyl]-carbamoyl-2-methyl-propyl)-carbamoyläthyl}-1,4,6-O-tris-trimethylsilyl-α,β-D-glucose, Sirup.

2-Isobutyroylamino-2-desoxy-3-O-[{-L-1-(D-1-carbamoyl-3-trimethylsilyl-carboxypropyl)-carbamoyl-propyl}-carbamoyl-methyl]-1,4,6-O-tris-trimethylsilyl-α,β-D-glucose, Sirup.

2-Acetamino-2-desoxy-3-O-[{L-1-(D-1-carbamoyl-3-trimethylsilyl-carboxypropyl)-carbamoyl-2-chloräthyl}-carbamoylmethyl]-1,4,6-O-tris-trimethylsilyl-α,β-D-glucose, Sirup.

2-Benzoylamino-2-desoxy-3-O-{D-1-[L-1-(D-1-carbamoyl-3-trimethylsilyl-carboxy-propyl)-carbamoyl-äthyl}-carbamoyl-äthyl]-1,4,6-O-tris-trimethylsilyl-α,β-D-glucose, Sirup.

Beispiel 4

Die genannten Trimethylsilylderivate können als Adjuvantien vorteilhaft auf folgende Arten angewendet werden:

1) Man löst 1 Teil Silylverbindung entweder zusammen mit 10 Teilen Eilecithin oder zusammen mit z. B. 8 Teilen Eilecithin und 2 Teilen Cholesterin in Chloroform, verdampft am Rotationsverdampfer zu einem Lipidfilm, den man mit einer Pufferlösung, z. B. phosphatgepufferter physologischer Kochsalzlösung pH = 7,2 vermengt. Unter leichter Kühlung behandelt man mit Ultraschall von 30–50 Watt etwa 3–5 Minuten, so dass die Innentemperatur 30° nicht übersteigt. Man erhält so eine Suspension von Lipidvesikeln, sogenannten Liposomen, mit einer sehr uniformen Grösse von etwa 1–3 μ Durchmesser. Leicht lipophile Antigene werden in wässriger Lösung dieser Suspension zugegeben und auf den Liposomen absorbiert. Die Antigene können jedoch auch vor der Beschallung zugegeben werden. So erhaltene Liposomen-Suspensionen können, i.p., s.c., i.d. oder i.m. gespritzt werden.

2) Man vermengt die erforderliche Menge der Silylverbindung mit Draceol, versetzt mit der wässrigen, gepufferten Lösung des Antigens und Arlacel und beschallt wie oben angegeben, bis man eine steife Wasser-in-Öl-Emulsion erhält, die gespritzt werden kann.

3) Die lipophilen Silylverbindungen können aus wässriger Suspension auch an Zellen, wie Erythrocyten oder Tumorzellen absorbiert werden, dabei können je nach Konzentration kleine Mengen Polyäthylenglykol 400 als Lösungsvermittler behilflich sein.

**Patentansprüche für die Vertragsstaaten: BE, CH, DE, FR, GB, IT, LU, NL, SE**

1. Silyl-Glucosaminderivate der Formel

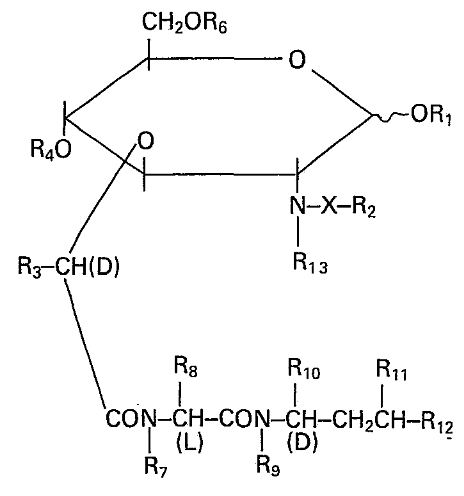

(I)

worin X eine Carbonylgruppe, $R_1$, $R_4$ und $R_6$ Triniederalkylsilyl, $R_2$ unsubstituiertes oder durch Hydroxy, Niederalkoxy, Triniederalkylsilyloxy, Niederalkanoyloxy, Halogen, Mercapto, Niederalkylmercapto, Triniederalkylsilylmercapto, Niederalkanoylmercapto, Carboxy, Niederalkoxycarbonyl und/oder Carbamoyl substituiertes Niederalkyl oder unsubstituiertes oder durch Niederalkyl, Hydroxy, Niederalkoxy, Niederalkanoyloxy, Triniederalkylsilyloxy, Niederalkylendioxy, Halogen, Amino und/oder Trifluoromethyl mono-, di- oder polysubstituiertes Phenyl, $R_3$, $R_7$ und $R_{13}$ Wasserstoff oder Niederalkyl, $R_8$ Wasserstoff, unsubstituiertes oder durch Hydroxy, Niederalkoxy, Triniederalkylsilyloxy, Niederalkanoyloxy, Halogen, Mercapto, Niederalkylmercapto, Triniederalkylsilylmercapto oder Niederalkanoylmercapto substituiertes Niederalkyl, Cycloalkyl mit 5 oder 6 Kohlenstoffatomen, Cycloalkyl-niederalkyl, dessen Cycloalkylrest 5 oder 6 Kohlenstoffatome enthält, einen unsubstituierten oder im Phenylteil durch Niederalkyl, Hydroxy, Niederalkoxy, Niederalkanoyloxy, Triniederalkylsilyloxy, Niederalkylendioxy, Halogen und/oder Trifluoromethyl mono-, di- oder polysubstituierten Phenyl- oder Phenylniederalkylrest, Heterocyclyl oder Heterocyclylniederalkyl mit jeweils 5 oder 6 Ringgliedern und ein oder zwei Stickstoffatomen im gegebenenfalls benzoannellierten heterocyclischen Ring, $R_7$ und $R_8$ zusammen auch Alkylen mit 3 oder 4 Kohlenstoffatomen, $R_9$ Wasserstoff oder Niederalkyl und die Reste $R_{10}$, $R_{11}$ und $R_{12}$ unabhängig voneinander Carboxy, Niederalkoxycarbonyl, Triniederalkylsilyloxycarbonyl oder unsubstituiertes oder durch Alkyl mit 1–18 C-Atomen oder einen unsubstituierten oder im Phenylteil durch Niederalkyl, Hydroxy, Niederalkoxy, Niederalkanoyloxy, Triniederalkylsilyloxy, Niederalkylendioxy, Halogen und/oder Trifluormethyl mono-, di- oder polysubstituierten Phenyl- oder Phenylniederalkylrest mono- oder disubstituiertes Carbamoyl oder durch Niederalkylen substituiertes Carbamoyl, $R_{10}$ auch durch Carbamoylmethyl oder L-1- oder D-1-Carbamoyläthyl substituiertes Carbamoyl und $R_{11}$ auch Wasserstoff bedeuten, wobei das vorstehend verwendete Präfix «Nieder-» einen Rest bis und mit 7 C-Atomen bezeichnet.

2. Silyl-Glucosaminderivate der in Anspruch 1 gegebenen Formel I gemäss Anspruch 1, worin X den Carbonylrest, $R_1$, $R_4$ und $R_6$ Triniederalkylsilyl, $R_2$ unsubstituiertes oder durch Halogen, Hydroxy oder Triniederalkylsilyloxy substituiertes Niederalkyl oder unsubstituiertes oder durch Niederalkyl, Niederalkoxy, Halogen oder Amino substituiertes Phenyl, $R_3$ Wasserstoff oder Alkyl mit 1–3 Kohlenstoffatomen, $R_7$, $R_9$ und $R_{13}$ Wasserstoff, $R_8$ Niederalkyl, Hydroxyniederalkyl, Triniederalkylsilyloxyniederalkyl, Mercaptoniederalkyl oder Triniederalkylsilylmercaptoniederalkyl, $R_7$ und $R_8$ zusammen auch einen Trimethylenrest, $R_{10}$ und $R_{12}$ unabhängig voneinander Carboxy, Niederalkoxycarbonyl, Triniederalkylsilyloxycarbonyl oder unsubstituiertes oder durch Alkyl mit 1–18 C-Atomen oder einen unsubstituierten oder im Phenylteil durch Niederalkyl, Hydroxy, Niederalkoxy, Niederalkanoyloxy, Triniederalkylsilyloxy, Niederalkylendioxy, Halogen und/oder Trifluormethyl mono-, di- oder polysubstituierten Phenyl- oder Phenylniederalkylrest mono- oder disubstituiertes Carbamoyl oder durch Niederalkylen substituiertes Carbamoyl, $R_{10}$ auch durch Carbamoylmethyl oder L-1- oder D-1-Carbamoyläthyl substituiertes Carbamoyl und $R_{11}$ Wasserstoff, Triniederalkylsilyloxycarbonyl oder einen Carboxyrest bedeuten.

3. Silyl-Glucosaminderivate der Formel I gemäss den Ansprüchen 1 oder 2, worin $R_3$ Wasserstoff darstellt.

4. Silyl-Glucosaminderivate der Formel I gemäss Anspruch 1, worin X den Carbonylrest, $R_1$, $R_4$ und $R_6$ Triniederalkylsilyl, $R_2$ Niederalkyl oder Phenyl, $R_3$ Wasserstoff oder Methyl, $R_7$, $R_9$ und $R_{13}$ Wasserstoff, $R_8$ Niederalkyl, Hydroxyniederalkyl, Triniederalkylsilyloxyniederalkyl, Mercaptomethyl oder Triniederalkylsilylmercaptomethyl, $R_7$ und $R_8$ zusammen auch den Trimethylenrest, $R_{10}$ und $R_{12}$ unabhängig voneinander Carboxy, Triniederalkylsilyloxycarbonyl oder Carbamoyl und $R_{11}$ Wasserstoff, Triniederalkylsilyloxycarbonyl oder einen Carboxyrest bedeuten.

5. Silyl-Glucosaminderivate gemäss einem der Ansprüche 1–4, worin $R_1$, $R_4$ und $R_6$ für Trimethylsilyl stehen.

6. Silyl-Glucosaminderivate der Formel I gemäss Anspruch 1, worin X den Carbonylrest, $R_1$, $R_4$ und $R_6$ Trimethylsilyl, $R_2$ Niederalkyl oder Phenyl, $R_3$ Wasserstoff oder Methyl, $R_7$, $R_9$ und $R_{13}$ Wasserstoff, $R_8$ Alkyl mit 1–3 Kohlenstoffatomen, Hydroxymethyl, 1-Hydroxyäthyl, Trimethylsilyloxymethyl, Mercaptomethyl oder Trimethylsilylmercaptomethyl, $R_{10}$ Carbamoyl, $R_{11}$ Wasserstoff und $R_{12}$ Carboxy, Trimethylsilyloxycarbonyl oder Carbamoyl bedeuten.

7. Die folgenden Verbindungen gemäss Anspruch 1: 2-Benzoylamino-2-desoxy-3-O-{[L-1-(D-1-carbamoyl-3-trimethylsilyloxycarbonyl-propyl)-carbamoyl-äthyl]-carbamoylmethyl}-1,4,6-O-tris-trimethylsilyl-α,β-D-glucose und 2-Benzoylamino-2-desoxy-3-O-{[L-1-(D-1-carbamoyl-3-carboxy-propyl)-carbamoyl-äthyl]-carbamoylmethyl}-1,4,6-O-tris-trimethylsilyl-α,β-D-glucose.

8. Die folgenden Verbindungen gemäss Anspruch 1: 2-Acetylamino-2-desoxy-3-O-{D-1-(L-1-[D-1-carbamoyl-3-trimethylsilyloxycarbonyl-propyl]-carbamoyl-äthyl-carbamoyl-äthyl-)}1,4,6-O-tris-trimethylsilyl-α,β-D-glucose und 2-Acetylamino-2-desoxy-3-O-{D-1-(L-1-[D-1-carbamoyl-3-carboxy-propyl]-carbamoyl-äthyl)-carbamoyl-äthyl}-1,4,6-O-tris-trimethylsilyl-α,β-D-glucose.

9. Die folgenden Verbindungen gemäss Anspruch 1: 2-Acetylamino-2-desoxy-3-O-{[L-1-(D-1-carbamoyl-3-trimethylsilyloxycarbonyl-propyl)-carbamoyl-äthyl]-carbamoylmethyl}-1,4,6-O-tris-trimethylsilyl-α,β-D-glucose und 2-Acetylamino-2-desoxy-3-O-{[L-1-D-1-carbamoyl-3-carboxy-propyl)-carbamoyl-äthyl]-carbamoylmethyl}-1,4,6-O-tris-trimethylsilyl-α,β-D-glucose.

10. Die folgenden Verbindungen gemäss Anspruch 1: 2-Propionylamino-2-desoxy-3-O-{[L-1-(D-1-carbamoyl-3-trimethylsilyloxycarbonyl-propyl)-carbamoyl-äthyl]-carbamoylmethyl}-1,4,6-O-tris-trimethylsilyl-α,β-D-glucose und 2-Propionylamino-2-desoxy-3-O-{[L-1-(D-1-carbamoyl-3-carboxy-propyl)-carbamoyl-äthyl]-carbamoylmethyl}-1,4,6-O-tris-trimethylsilyl-α,β-D-glucose.

11. Verbindungen der Formel I gemäss einem der Ansprüche 1–10 mit immunomodulierender Wirkung.

12. Pharmazeutische Präparate, die eine Verbindung gemäss einem der Patentansprüche 1–10 zusammen mit einem pharmazeutisch verwendbaren Trägermaterial enthalten.

13. Verbindungen der Formel I gemäss einem der Ansprüche 1–10 zur Anwendung in einem Verfahren zur therapeutischen Behandlung des menschlichen oder tierischen Körpers.

14. Verwendung einer der in den Ansprüchen 1–10 genannten Verbindungen zur Herstellung eines Immunisierungsmittels bzw. Impfstoffes auf nicht-chemischem Wege.

15. Verwendung einer der in den Ansprüchen 1–10 genannten Verbindungen als Adjuvantien in Mischung mit Impfstoffen.

16. Verfahren zur Herstellung der in den Patentansprüchen 1–10 beanspruchten Verbindungen, dadurch gekennzeichnet, dass man in an sich bekannter Weise eine Verbindung der Formel

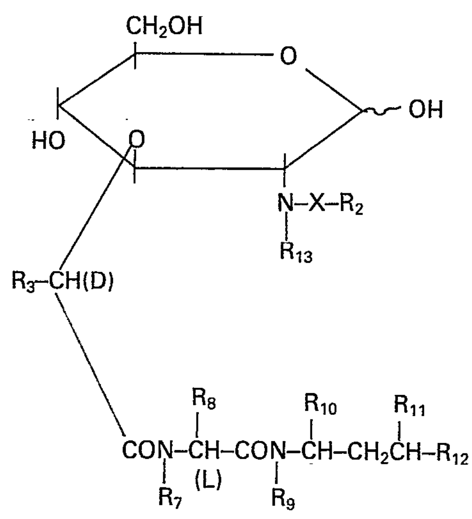

(II)

worin die Substituenten wie in einem der Ansprüche 1–8 definiert sind, mit einem einen Triniederalkylsilylrest einführenden Silylierungsmittel umsetzt und erhaltene Silylester der in Anspruch 1 aufgeführten Formel I gegebenenfalls mit Wasser zu den Silyläthern der Formel I aufspaltet.

17. Die nach dem Verfahren des Anspruchs 16 erhältlichen Verbindungen.

**Patentansprüche für den Vertragsstaat AT**

1. Verfahren zur Herstellung von Silyl-Glucosaminderivaten der Formel

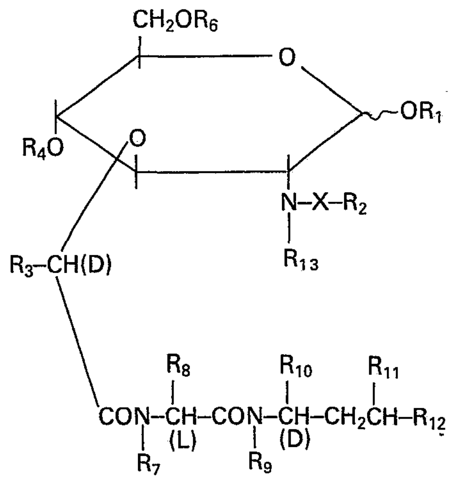

(I)

worin X eine Carbonylgruppe, $R_1$, $R_4$ und $R_6$ Triniederalkylsilyl, $R_2$ unsubstituiertes oder durch Hydroxy, Niederalkoxy, Triniederalkylsilyloxy, Niederalkanoyloxy, Halogen, Mercapto, Niederalkylmercapto, Triniederalkylsilylmercapto, Niederalkanoylmercapto, Carboxy, Niederalkoxycarbonyl und/oder Carbamoyl substituiertes Niederalkyl oder unsubstituiertes oder durch Niederalkyl, Hydroxy, Niederalkoxy, Niederalkanoyloxy, Triniederalkylsilyloxy, Niederalkylendioxy, Halogen, Amino und/oder Trifluoromethyl mono-, di- oder polysubstituiertes Phenyl, $R_3$, $R_7$ und $R_{13}$ Wasserstoff oder Niederalkyl, $R_8$ Wasserstoff, unsubstituiertes oder durch Hydroxy, Niederalkoxy, Triniederalkylsilyloxy, Niederalkanoyloxy, Halogen, Mercapto, Niederalkylmercapto, Triniederalkylsilylmercapto oder Niederalkanoylmercapto substituiertes Niederalkyl, Cycloalkyl mit 5 oder 6 Kohlenstoffatomen, Cycloalkyl-niederalkyl, dessen Cycloalkylrest 5 oder 6 Kohlenstoffatome enthält, einen unsubstituierten oder im Phenylteil durch Niederalkyl, Hydroxy, Niederalkoxy, Niederalkanoyloxy, Triniederalkylsilyloxy, Niederalkylendioxy, Halogen und/oder Trifluormethyl, mono-, di- oder polysubstituierten Phenyl- oder Phenylniederalkylrest, Heterocyclyl- oder Heterocyclylniederalkyl mit jeweils 5 oder 6 Ringgliedern und ein oder zwei Stickstoffatomen im gegebenenfalls benzoannellierten heterocyclischen Ring, $R_7$ und $R_8$ zusammen auch Alkylen mit 3 oder 4 Kohlenstoffatomen, $R_9$ Wasserstoff oder Niederalkyl und die Reste $R_{10}$, $R_{11}$ und $R_{12}$ unabhängig voneinander Carboxy, Niederalkoxycarbonyl, Triniederalkylsilyloxycarbonyl oder unsubstituiertes oder durch Alkyl mit 1–18 C-Atomen oder einen unsubstituierten oder im Phenylteil durch Niederalkyl, Hydroxy, Niederalkoxy, Niederalkanoyloxy, Triniederalkylsilyloxy, Niederalkylendioxy, Halogen und/oder Trifluoromethyl mono-, di- oder polysubstituierten Phenyl- oder Phenylniederalkylrest mono- oder disubstituiertes Carbamoyl oder durch Niederalkylen substituiertes Carbamoyl, $R_{10}$ auch durch Carbamoylmethyl oder L-1- oder D-1-Carbamoyläthyl substituiertes Carbamoyl und $R_{11}$ auch Wasserstoff bedeuten, wobei das vor- und nachstehend verwendete Präfix «Nieder-» einen Rest bis und mit 7 C-Atomen bezeichnet, dadurch gekennzeichnet, dass man in an sich bekannter Weise eine Verbindung der Formel II

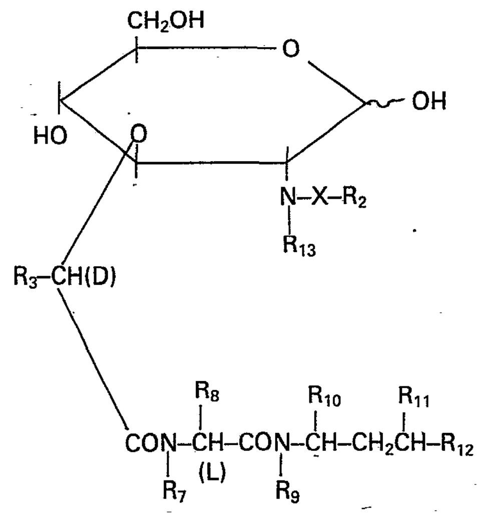

(II)

worin die Substituenten wie oben definiert sind, mit einem einen Triniederalkylsilylrest einführenden Silylierungsmittel umsetzt und erhaltene Silylester der Formel I gegebenenfalls mit Wasser in die Silyläther der Formel I überführt.

2. Verfahren nach Anspruch 1, dadurch gekennzeichnet, dass man Silyl-Glucosaminderivate der in Patentanspruch 1 gegebenen Formel I herstellt, worin X den Carbonylrest, $R_1$, $R_4$ und $R_6$ Triniederalkylsilyl, $R_2$ unsubstituiertes oder durch Halogen, Hydroxy oder Triniederalkylsilyloxy substituiertes Niederalkyl oder unsubstituiertes oder durch Niederalkyl, Niederalkoxy, Halogen oder Amino substituiertes Phenyl, $R_3$ Wasserstoff oder Alkyl mit 1–3 Kohlenstoffatomen, $R_7$, $R_9$ und $R_{13}$ Wasserstoff, $R_8$ Niederalkyl, Hydroxyniederalkyl, Triniederalkylsilyloxyniederalkyl, Mercaptoniederalkyl oder Triniederalkylsilylmercaptoniederalkyl, $R_7$ und $R_8$ zusammen auch einen Trimethylenrest, $R_{10}$ und $R_{12}$ unabhängig voneinander Carboxy, Niederalkoxycarbonyl, Triniederalkylsilyloxycarbonyl oder unsubstituiertes oder durch Alkyl mit 1–18 C-Atomen oder einen unsubstituierten oder im Phenylteil durch Niederalkyl, Hydroxy, Niederalkoxy, Niederalkanoyloxy, Triniederalkylsilyloxy, Niederalkylendioxy, Halogen und/oder Trifluoromethyl mono-, di- oder polysubstituierten Phenyl- oder Phenylniederalkylrest mono- oder disubstituiertes Carbamoyl oder durch Niederalkylen substituiertes Carbamoyl, $R_{10}$ auch durch Carbamoylme-

thyl oder L-1- oder D-1-Carbamoyläthyl substituiertes Carbamoyl und $R_{11}$ Wasserstoff, Triniederalkylsilyloxycarbonyl oder einen Carboxyrest bedeuten.

3. Verfahren nach Anspruch 1 oder 2, dadurch gekennzeichnet, dass man Silyl-Glucosaminderivate herstellt, worin in der gegebenen Formel I $R_3$ Wasserstoff darstellt.

4. Verfahren nach einem der Ansprüche 1–3, dadurch gekennzeichnet, dass man Silyl-Glucosaminderivate der in Patentanspruch 1 gegebenen Formel I herstellt, worin X den Carbonylrest, $R_1$, $R_4$ und $R_6$ Triniederalkylsilyl, $R_2$ Niederalkyl oder Phenyl, $R_3$ Wasserstoff oder Methyl, $R_7$, $R_9$ und $R_{13}$ Wasserstoff, $R_8$ Niederalkyl, Hydroxyniederalkyl, Triniederalkylsilyloxyniederalkyl, Mercaptomethyl oder Triniederalkylsilylmercaptomethyl, $R_7$ und $R_8$ zusammen auch den Trimethylenrest, $R_{10}$ und $R_{12}$ unabhängig voneinander Carboxy, Triniederalkylsilyloxycarbonyl oder Carbamoyl und $R_{11}$ Wasserstoff, Triniederalkylsilyloxycarbonyl oder einen Carboxyrest bedeuten.

5. Verfahren nach einem der Ansprüche 1–4, dadurch gekennzeichnet, dass man Verbindungen der in Anspruch 1 gegebenen Formel I herstellt, worin $R_1$, $R_4$ und $R_6$ für Trimethylsilyl stehen.

6. Verfahren nach einem der Ansprüche 1–4, dadurch gekennzeichnet, dass man Silyl-Glucosaminderivate der in Patentanspruch 1 gegebenen Formel I herstellt, worin X den Carbonylrest, $R_1$, $R_4$ und $R_6$ Trimethylsilyl, $R_2$ Niederalkyl oder Phenyl, $R_3$ Wasserstoff oder Methyl, $R_7$, $R_9$ und $R_{13}$ Wasserstoff, $R_8$ Alkyl mit 1–3 Kohlenstoffatomen, Hydroxymethyl, 1-Hydroxy-äthyl, Trimethylsilyloxymethyl, Mercaptomethyl oder Trimethylsilylmercaptomethyl, $R_{10}$ Carbamoyl, $R_{11}$ Wasserstoff und $R_{12}$ Carboxy, Trimethylsilyloxycarbonyl oder Carbamoyl bedeuten.

7. Verfahren nach Anspruch 1, dadurch gekennzeichnet, dass man 2-Benzoylamino-2-desoxy-3-O-{[L-1-(D-1-carbamoyl-3-trimethylsilyloxycarbonyl-propyl)-carbamoyl-äthyl]-carbamoylmethyl}-1,4,6-O-tris-trimethylsilyl-α,β-D-glucose oder 2-Benzoylamino-2-desoxy-3-O-{[L-1-(D-1-carbamoyl-3-carboxy-propyl)-carbamoyl-äthyl]-carbamoylmethyl}-1,4,6-O-tris-trimethylsilyl-α,β-D-glucose herstellt.

8. Verfahren nach Anspruch 1, dadurch gekennzeichnet, dass man 2-Acetylamino-2-desoxy-3-O-{D-1-(L-1-D-1-carbamoyl-3-trimethylsilyloxycarbonyl-propyl]-carbamoyl-äthyl)-carbamoyl-äthyl}-1,4,6-O-tris-trimethylsilyl-α,β-D-glucose oder 2-Acetylamino-2-desoxy-3-O-{D-1-(L-1-[D-1-carbamoyl-3-carboxy-propyl]-carbamoyl-äthyl)-carbamoyl-äthyl}-1,4,6-O-tris-trimethylsilyl-α,β-D-glucose herstellt.

9. Verfahren nach Anspruch 1, dadurch gekennzeichnet, dass man 2-Acetylamino-2-desoxy-3-O-{[L-1-(D-1-carbamoyl-3-trimethylsilyloxycarbonyl-propyl)-carbamoyl-äthyl]-carbamoylmethyl}-1,4,6-O-tris-trimethylsilyl-α,β-D-glucose oder 2-Acetylamino-2-desoxy-3-O-{[L-1-D-1-carbamoyl-3-carboxy-propyl)-carbamoyl-äthyl]-carbamoyl-methyl}-1,4,6-O-tris-trimethylsilyl-α,β-D-glucose herstellt.

10. Verfahren nach Anspruch 1, dadurch gekennzeichnet, dass man 2-Propionylamino-2-desoxy-3-O-{[L-1-(D-1-carbamoyl-3-trimethylsilyloxycarbonyl-propyl)-carbamoyl-äthyl]-carbamoylmethyl}-1,4,6-O-tris-trimethylsilyl-α,β-D-glucose oder 2-Propionylamino-2-desoxy-3-O-{[L-1-(D-1-carbamoyl-3-carboxy-propyl)-carbamoyl-äthyl]-carbamoylmethyl}-1,4,6-O-tris-trimethylsilyl-α,β-D-glucose herstellt.

11. Verfahren nach einem der Ansprüche 1–10, dadurch gekennzeichnet, dass man als Silylierungsmittel, Triniederalkylsilylhalogenide, Hexaniederalkyldisilazan, Triniederalkylsilylamin, Triniederalkylsilyldiäthylamin, Triniederalkylsilylacetamid, Bis-(triniederalkylsilyl)-acetamide oder Triniederalkylsilyl-sulfamide verwendet.

12. Verfahren zur Herstellung eines pharmazeutischen Präparates, dadurch gekennzeichnet, dass man eine Verbindung der Formel I gemäss Anspruch 1 mit einem pharmazeutisch verwendbaren Trägermaterial mischt.

**Claims for the Contracting States: BE, CH, DE, FR, GB, IT, LU, NL, SE**

1. A silyl glucosamine derivative of the formula

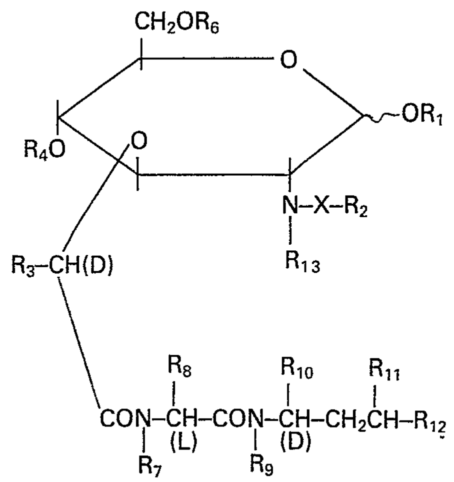

(I)

wherein X is a carbonyl group, $R_1$, $R_4$ and $R_6$ are tri-lower alkylsilyl, $R_2$ is lower alkyl which is unsubstituted or substituted by hydroxy, lower alkoxy, tri-lower alkylsilyloxy, lower alkanoyloxy, halogen, mercapto, lower alkylmercapto, tri-lower alkylsilylmercapto, lower alkanoylmercapto, carboxyl, lower alkoxycarbonyl and/or carbamoyl, or is phenyl which is unsubstituted or mono-, di- or polysubstituted by lower alkyl, hydroxyl, lower alkoxy, lower alkanoyloxy, tri-lower alkylsilyloxy, lower alkylenedioxy, halogen, amino and/or trifluoromethyl; $R_3$, $R_7$ and $R_{13}$ are hydrogen or lower alkyl, $R_8$ is hydrogen, lower alkyl which is unsubstituted or substituted by hydroxyl, lower alkoxy, tri-lower alkylsilyloxy, lower alkanoyloxy, halogen, mercapto, lower alkylmercapto, tri-lower alkylsilylmercapto or lower alkanoylmercapto, or is $C_5$–$C_6$ cycloalkyl, cycloalkyl-lower alkyl containing 5 or 6 carbon atoms in the cycloalkyl moiety, a phenyl or phenyl-lower alkyl radical which is unsubstituted or mono-, di- or polysubstituted in the

phenyl moiety by lower alkyl, hydroxyl, lower alkoxy, lower alkanoyloxy, tri-lower alkylsilyloxy, lower alkylenedioxy, halogen and/or trifluoromethyl, or is heterocyclyl or heterocyclyl-lower alkyl each containing 5 or 6 ring members and one or two-nitrogen atoms in the heterocyclic ring which may or may not be fused to a benzene ring, and $R_7$ and $R_8$, when taken together, are also $C_3$–$C_4$ alkylene; $R_9$ is hydrogen or lower alkyl, and the radicals $R_{10}$, $R_{11}$ and $R_{12}$ are each independently carboxyl, lower alkoxycarbonyl, tri-lower alkylsilyloxycarbonyl or carbamoyl which is unsubstituted or mono- or disubstituted by $C_1$–$C_{18}$ alkyl or by a phenyl or phenyl-lower alkyl, radical which is unsubstituted or mono-, di- or polysubstituted in the phenyl moiety by lower alkyl, hydroxyl, lower alkoxy, lower alkanoyloxy, tri-lower alkylsilyloxy, lower alkylenedioxy, halogen and/or trifluoromethyl, or is carbamoyl substituted by lower alkylene, and $R_{10}$ is also carbamoyl substituted by carbamoylmethyl or L-1- or D-1-carbamoylethyl and $R_{11}$ is also hydrogen, the prefix «lower» used to qualify the radicals cited above denoting a radical containing up to and including 7 carbon atoms.

2. A silyl glucosamine derivative of the formula I according to claim 1, wherein X is the carbonyl radical, $R_1$, $R_4$ and $R_6$ are tri-lower alkylsilyl, $R_2$ is lower alkyl which is unsubstituted or substituted by halogen, hydroxyl or tri-lower alkylsilyloxy, or is phenyl which is unsubstituted or substituted by lower alkyl, lower alkoxy, halogen or amino, $R_3$ is hydrogen or $C_1$—$C_3$ alkyl, $R_7$, $R_9$ and $R_{13}$ are hydrogen, $R_8$ is lower alkyl, hydroxy-lower alkyl, tri-lower alkylsilyloxy-lower alkyl, mercapto-lower alkyl or tri-lower alkylsilylmercapto-lower alkyl, and $R_7$ and $R_8$, when taken together, are also a trimethylene radical, $R_{10}$ and $R_{11}$ are each independently carboxyl, lower alkoxycarbonyl, tri-lower alkylsilyloxycarbonyl or carbamoyl which is unsubstituted or mono- or disubstituted by $C_1$–$C_{18}$ alkyl or by a phenyl or phenyl-lower alkyl radical which is unsubstituted or mono-, di- or polysubstituted in the phenyl moiety by lower alkyl, hydroxyl, lower alkoxy, lower alkanoyloxy, tri-lower alkylsilyloxy, lower alkylenedioxy, halogen and/or trifluoromethyl, or is carbamoyl substituted by lower alkylene, and $R_{10}$ is also carbamoyl substituted by carbamoylmethyl or L-1- or D-1-carbamoylethyl, and $R_{11}$ is hydrogen, tri-lower alkylsilyloxycarbonyl or a carboxyl group.

3. A silyl glucosamine derivative of the formula I according to either of claims 1 or 2, wherein $R_3$ is hydrogen.

4. A silyl glucosamine derivative of the formula I, wherein X is the carbonyl radical, $R_1$, $R_4$ and $R_6$ are tri-lower alkylsilyl, $R_2$ is lower alkyl or phenyl, $R_3$ is hydrogen or methyl, $R_7$, $R_9$ and $R_{13}$ are hydrogen, $R_8$ is lower alkyl, hydroxy-lower alkyl, tri-lower alkylsilyloxy-lower alkyl, mercaptomethyl or tri-lower alkylsilylmercaptomethyl, $R_7$ and $R_8$, when taken together, are also the trimethylene radical, $R_{10}$ and $R_{12}$ are each independently carboxyl, tri-lower alkylsilyloxycarbonyl or carbamoyl, and $R_{11}$ is hydrogen, tri-lower alkylsilyloxycarbonyl or carbamoyl, and $R_{11}$ is hydrogen, tri-lower alkylsilyloxycarbonyl or a carboxyl group.

5. A silyl glucosamine derivative according to any one of claims 1 to 4, wherein $R_1$, $R_4$ and $R_6$ are trimethylsilyl.

6. A silyl glucosamine derivative of the formula I according to claim 1, wherein X is the carbonyl radical, $R_1$, $R_4$ and $R_6$ are trimethylsilyl, $R_2$ is lower alkyl or phenyl, $R_3$ is hydrogen or methyl, $R_7$, $R_9$ and $R_{13}$ are hydrogen, $R_8$ is $C_1$–$C_3$ alkyl, hydroxymethyl, 1-hydroxyethyl, trimethylsilyloxymethyl, mercaptomethyl or trimethylsilylmercaptomethyl, $R_{10}$ is carbamoyl, $R_{11}$ is hydrogen and $R_{12}$ is carboxyl, trimethylsilyloxycarbonyl or carbamoyl.

7. 2-Benzoylamino-2-desoxy-3-O-{[L-1-D-1-carbamoyl-3-trimethylsilyloxycarbonylpropyl)carbamoylethyl]carbamoylmethyl}-1,4,6-O-tris-trimethylsilyl-α,β-D-glucose and 2-benzoylamino-2-desoxy-3-O-{[L-1-D-1-carbamoyl-3-carboxypropyl)-carbamoylethyl]carbamoylmethyl}-1,4,6-O-tris-trimethylsilyl-α,β-D-glucose, according to claim 1.

8. 2-Acetylamino-2-desoxy-3-O-{D-1-(L-1-carbamoyl-3-trimethylsilyloxycarbonylpropyl]carbamoylethyl)carbamoylethyl}-1,4,6-O-tris-trimethylsilyl-α,β-D-glucose and 2-acetylamino-2-desoxy-3-O-{D-1-(L-1-[D-1-carbamoyl-3-carboxypropyl]carbamoylethyl)carbamoylethyl}-1,4,6-O-tris-trimethylsilyl-α,β-D-glucose, according to claim 1.

9. 2-Acetylamino-2-desoxy-3-O-{]L-1-(D-1-carbamoyl-3-trimethylsilyloxycarbonylpropyl)carbamoylethyl]carbamoylmethyl}-1,4,6-O-tris-trimethylsilyl-α,β-D-glucose and 2-acetylamino-2-desoxy-3-O-{[L-1-D-1-carbamoyl-3-carboxypropyl)carbamoylethyl]carbamoylmethyl}-1,4,6-O-tris-trimethylsilyl-α,β-D-glucose, according to claim 1.

10. 2-Propionylamino-2-desoxy-3-O-{[L-1-(D-1-carbamoyl-3-trimethylsilyloxycarbonylpropyl)carbamoylethyl]carbamoylmethyl}-1,4,6-O-tris-trimethylsilyl-α,β-D-glucose and 2-propionylamino-2-desoxy-3-O-{[L-1-(D-1-carbamoyl-3-carboxypropyl)carbamoylethyl]carbamoylmethyl}-1,4,6-O-tris-trimethylsilyl-α,β-D-glucose, according to claim 1.

11. A compound of the formula I according to any one of claims 1 to 10 having immunomodulating action.

12. A pharmaceutical preparation which contains a compound as claimed in any one of claims 1 to 10, together with a pharmaceutically acceptable carrier.

13. A compound of the formula I according to any one of claims 1 to 10 for use in a therapeutic method of treating the human or animal body.

14. Use of a compound as claimed in any one of claims 1 to 10 for the preparation of an immunising agent or vaccine by non-chemical means.

15. Use of a compound as claimed in any one of claims 1 to 10 as an adjuvant in admixture with a vaccine.

16. A process for the preparation of a compound as claimed in any one of claims 1 to 10, which

comprises reacting, in a manner known per se, a compound of the formula

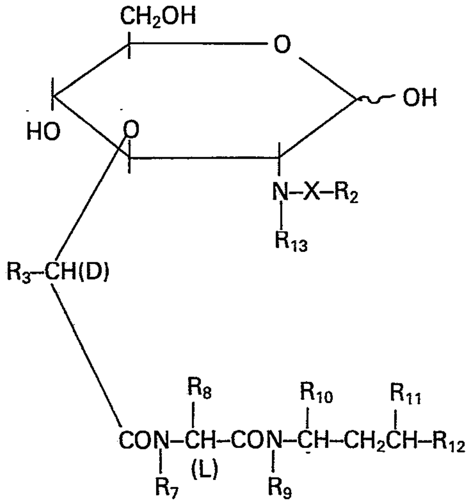

(II)

wherein the substituents are as defined in any one of claims 1 to 8, with a silylating agent which introduces a tri-lower alkylsilyl radical, and cleaving a resultant silyl ester of the formula I as indicated in claim 1, optionally with water, to give a silyl ether of the formula I.

17. A compound obtainable by the process as claimed in claim 16.

**Claims for the Contracting State: AT**

1. A process for the preparation of a silyl glucosamine derivative of the formula

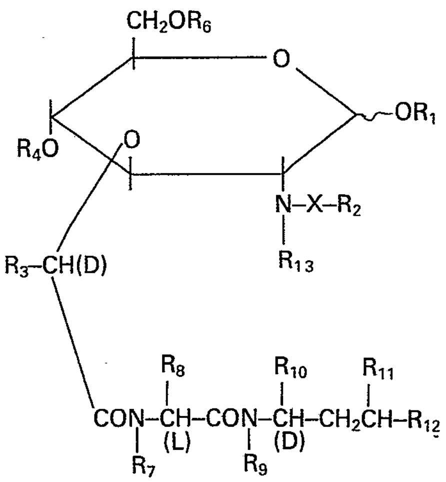

(I)

wherein X is a carbonyl group, $R_1$, $R_4$ and $R_6$ are tri-lower alkylsilyl, $R_2$ is lower alkyl which is unsubstituted or substituted by hydroxy, lower alkoxy, tri-lower alkylsilyloxy, lower alkanoyloxy, halogen, mercapto, lower alkylmercapto, tri-lower alkylsilylmercapto, lower alkanoylmercapto, carboxyl, lower alkoxycarbonyl and/or carbamoyl, or is phenyl which is unsubstituted or mono-, di- or polysubstituted by lower alkyl, hydroxyl, lower alkoxy, lower alkanoyloxy, tri-lower alkylsilyloxy, lower alkylenedioxy, halogen, amino and/or trifluoromethyl; $R_3$, $R_7$ and $R_{13}$ are hydrogen or lower alkyl, $R_8$ is hydrogen, lower alkyl which is unsubstituted or substituted by hydroxyl, lower alkoxy, tri-lower alkylsilyloxy, lower alkanoyloxy, halogen, mercapto, lower alkylmercapto, tri-lower alkylsilylmercapto or lower alkanoylmercapto, or is $C_5$–$C_6$ cycloalkyl, cycloalkyl-lower alkyl containing 5 or 6 carbon atoms in the cycloalkyl moiety, a phenyl or phenyl-lower alkyl radical which is unsubstituted or mono-, di- or polysubstituted in the phenyl moiety by lower alkyl, hydroxyl, lower alkoxy, lower alkanoyloxy, tri-lower alkylsilyloxy, lower alkylenedioxy, halogen and/or trifluoromethyl, or is heterocyclyl or heterocyclyl-lower alkyl each containing 5 or 6 ring members and one or two-nitrogen atoms in the heterocyclic ring which may or may not be fused to a benzene ring, and $R_7$ and $R_8$, when taken together, are also $C_3$–$C_4$ alkylene; $R_9$ is hydrogen or lower alkyl, and the radicals $R_{10}$, $R_{11}$ and $R_{12}$ are each independently carboxyl, lower alkoxycarbonyl, tri-lower alkylsilyloxycarbonyl or carbamoyl which is unsubstituted or mono- or disubstituted by $C_1$–$C_{18}$ alkyl or by a phenyl or phenyl-lower alkyl radical which is unsubstituted or mono-, di- or polysubstituted in the phenyl moiety by lower alkyl, hydroxyl, lower alkoxy, lower alkanoyloxy, tri-lower alkylsilyloxy, lower alkylenedioxy, halogen and/or trifluoromethyl, or is carbamoyl substituted by lower alkylene, and $R_{10}$ is also carbamoyl substituted by carbamoylmethyl or L-1- or D-1-carbamoylethyl, and $R_{11}$ is also hydrogen, the prefix «lower» used to qualify the radicals cited above denoting a radical containing up to and including 7 carbon atoms, which comprises reacting, in a manner known per se, a compound of the formula

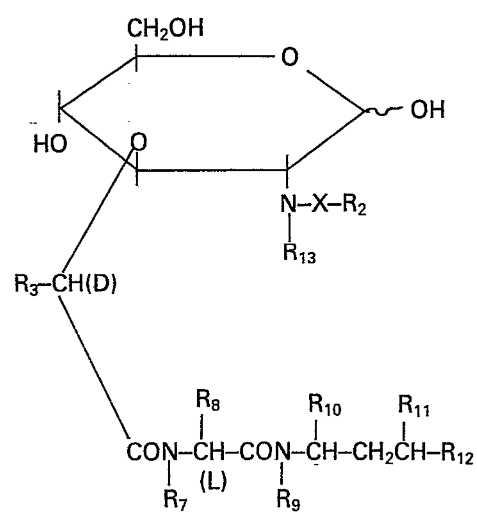

(II)

wherein the substituents are as defined in any one of claims 1 to 8, with a silylating agent which introduces a tri-lower alkylsilyl radical, and converting a resultant silyl ester of the formula I as indicated in claim 1, optionally with water, to give a silyl ether of the formula I.

2. A process according to claim 1 for the preparation of a silyl glucosamine derivative of the formula I as indicated in claim 1, wherein X is the

carbonyl radical, $R_1$, $R_4$ and $R_6$ are tri-lower alkylsilyl, $R_2$ is lower alkyl which is unsubstituted or substituted by halogen, hydroxyl or tri-lower alkylsilyloxy, or is phenyl which is unsubstituted or substituted by lower alkyl, lower alkoxy, halogen or amino, $R_3$ is hydrogen or $C_1$–$C_3$ alkyl, $R_7$, $R_9$ and $R_{13}$ are hydrogen, $R_8$ is lower alkyl, hydroxy-lower alkyl, tri-lower alkylsilyloxy-lower alkyl, mercapto-lower alkyl or tri-lower alkylsilylmercapto-lower alkyl, and $R_7$ and $R_8$, when taken together, are also a trimethylene radical, $R_{10}$ and $R_{11}$ are each independently carboxyl, lower alkoxycarbonyl, tri-lower alkylsilyloxycarbonyl or carbamoyl which is unsubstituted or mono- or disubstituted by $C_1$–$C_{18}$ alkyl or by a phenyl or phenyl-lower alkyl radical which is unsubstituted or mono- di- or polysubstituted in the phenyl moiety by lower alkyl, hydroxyl, lower alkoxy, lower alkanoyloxy, tri-lower alkylsilyloxy, lower alkylenedioxy, halogen and/or trifluoromethyl, or is carbamoyl substituted by lower alkylene, and $R_{10}$ is also carbamoyl substituted by carbamoylmethyl or L-1- or D-1-carbamoylethyl, and $R_{11}$ is hydrogen, tri-lower alkylsilyloxycarbonyl or a carboxyl group.

3. A process according to either of claims 1 or 2 for the preparation of a silyl glucosamine derivative, wherein $R_3$ in formula I is hydrogen.

4. A process according to any one of claims 1 to 3 for the preparation of a silyl glucosamine derivative of the formula I as indicated in claim 1, wherein X is the carbonyl radical, $R_1$, $R_4$ and $R_6$ are tri-lower alkylsilyl, $R_2$ is lower alkyl or phenyl, $R_3$ is hydrogen or methyl, $R_7$, $R_9$ and $R_{13}$ are hydrogen, $R_8$ is lower alkyl, hydroxy-lower alkyl, tri-lower alkylsilyloxy-lower alkyl, mercaptomethyl or tri-lower alkylsilylmercaptomethyl, $R_7$ and $R_8$, when taken together, are also the trimethylene radical, $R_{10}$ and $R_{12}$ are each independently carboxyl, tri-lower alkylsilyloxycarbonyl or carbamoyl and $R_{11}$ is hydrogen, tri-lower alkylsilyloxycarbonyl or carbamoyl, and $R_{11}$ is hydrogen, tri-lower alkylsilyloxycarbonyl or a carboxyl group.

5. A process according to any one of claims 1 to 4 for the preparation of a compound of the formula I as indicated in claim 1, wherein $R_1$, $R_4$ and $R_6$ are trimethylsilyl.

6. A process according to any one of claims 1 to 4 for the preparation of a silyl glucosamine derivative of the formula I as indicated in claim 1, wherein X is the carbonyl radical, $R_1$, $R_4$ and $R_6$ are trimethylsilyl, $R_2$ is lower alkyl or phenyl, $R_3$ is hydrogen or methyl, $R_7$, $R_9$ and $R_{13}$ are hydrogen, $R_8$ is $C_1$–$C_3$ alkyl, hydroxymethyl, 1-hydroxyethyl, trimethylsilyloxymethyl, mercaptomethyl, or trimethylsilylmercaptomethyl, $R_{10}$ is carbamoyl, $R_{11}$ is hydrogen and $R_{12}$ is carboxyl, trimethylsilyloxycarbonyl or carbamoyl.

7. A process according to claim 1 for the preparation of 2-benzoylamino-2-desoxy-3-O-{[L-1-D-1-carbamoyl-3-trimethylsilyloxycarbonylpropyl)carbamoylethyl]carbamoylmethyl}-1,4,6-O-tris-trimethylsilyl-α,β-D-glucose or 2-benzoylamino-2-desoxy-3-O-{[11L-1-D-1-carbamoyl-3-carboxypropyl)-carbamoylethyl]carbamoylmethyl}-1,4,6-O-tris-trimethylsilyl-α,β-D-glucose.

8. A process according to claim 1 for the preparation of 2-acetylamino-2-desoxy-3-O-{D-1-(L-1-carbamoyl-3-trimethylsilyloxycarbonylpropyl]carbamoylethyl)carbamoylethyl}-1,4,6-O-tris-trimethylsilyl-α,β-D-glucose or 2-acetylamino-2-desoxy-3-O-{D-1-(L-1-(D-1-carbamoyl-3-carboxypropyl]carbamoylethyl)carbamoylethyl}-1,4,6-O-tris-trimethylsilyl-α,β-D-glucose.

9. A process according to claim 1 for the preparation of 2-acetylamino-2-desoxy-3-O-{[L-1-(D-1-carbamoyl-3-trimethylsilyloxycarbonylpropyl)carbamoylethyl]carbamoylmethyl}-1,4,6-O-tris-trimethylsilyl-α,β-D-glucose or 2-acetylamino-2-desoxy-3-O-{[L-1-D-1-carbamoyl-3-carboxypropyl)carbamoylethyl]carbamoylmethyl}-1,4,6-O-tris-trimethylsilyl-α,β-D-glucose.

10. A process according to claim 1 for the preparation of 2-propionylamino-2-desoxy-3-O-{[L-1-(D-1-carbamoyl-3-trimethylsilyloxycarbonylpropyl)carbamoylethyl]carbamoylmethyl}-1,4,6-O-tris-trimethylsilyl-α,β-D-glucose or 2-propionylamino-2-desoxy-3-O-{[L-1-(D-1-carbamoyl-3-carboxypropyl)carbamoylethyl]carbamoylmethyl}-1,4,6-O-tris-trimethylsilyl-α,β-D-glucose.

11. A process according to any one of claims 1 to 10, wherein the silylating agent is a tri-lower alkylsilyl halide, a hexa-lower alkyl disilazane, a tri-lower alkylsilylamine, a tri-lower alkylsilyl diethylamine, a tri-lower alkylsilylacetamide, a bis(tri-lower alkylsilyl)-acetamide or a tri-lower alkylsilylsulfamide.

12. A process for the production of a pharmaceutical preparation which comprises mixing a compound as claimed in claim 1 with a pharmaceutically acceptable carrier.

**Revendications pour les Etats contractants: BE, CH, DE, FR, GB, IT, LU, NL, SE**

1. Dérivés silylés de glucosamines de formule:

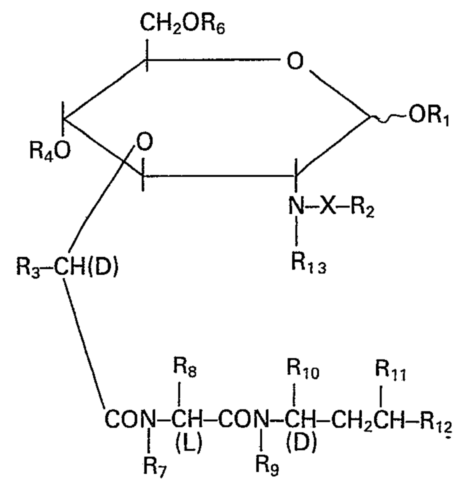

(I)

dans laquelle:

X représente un groupe carbonyle,

$R_1$, $R_4$ et $R_6$ représentent des groupes tri-(alkyle inférieur)-silyles,

$R_2$ représente un groupe alkyle inférieur non substitué ou substitué par des groupes hydroxy, alcoxy inférieurs, tri-(alkyle inférieur)-

silyloxy, alcanoyloxy inférieurs, des halogènes, des groupes mercapto, alkylmercapto inférieurs, tri-(alkyle inférieur)-silylmercapto, alcanoylmercapto inférieurs, carboxy, alcoxycarbonyle inférieurs et/ou carbamoyles ou un groupe phényle non substitué ou mono-, di-, ou poly-substitué par des groupes alkyles inférieurs, hydroxy, alcoxy inférieurs, alcanoyloxy inférieurs, tri-(alkyle inférieur)-silyloxy, alkylène inférieur-dioxy, des halogènes, des groupes amino et/ou trifluorométhyle,

$R_3$, $R_7$ et $R_{13}$ représentent l'hydrogène ou des groupes alkyles inférieurs,

$R_8$ représente l'hydrogène, un groupe alkyle inférieur non substitué ou substitué par des groupes hydroxy, alcoxy inférieurs, tri-(alkyle inférieur) silyloxy, alcanoyloxy inférieurs, des halogènes, des groupes mercapto, alkylmercapto inférieurs, tri-(alkyle inférieur)-silylmercapto ou alcanoylmercapto inférieurs, un groupe cycloalkyle en $C_5$–$C_6$, un groupe cycloalkyl-inférieur dont la partie cycloalkyle contient 5 ou 6 atomes de carbone, un groupe phényle ou phénylalkyle inférieur non substitué ou mono-, di-, ou poly-substitué dans la partie phényle par des groupes alkyles inférieurs, hydroxy, alcoxy inférieurs, alcanoyloxy inférieurs, tri-(alkyle inférieur)-silyloxy, alkylène-dioxy inférieurs, des halogènes et/ou des groupes trifluorométhyles, un groupe hétérocyclyle ou hétérocyclyl-alkyle inférieur contenant chacun 5 ou 6 chaînons cycliques et un ou deux atomes d'azote dans le noyau hétérocyclique éventuellement condensé sur un noyau benzénique,

$R_7$ et $R_8$ peuvent également former un groupe alkylène en $C_3$ ou $C_4$,

$R_9$ représente l'hydrogène ou un groupe alkyle inférieur, et les symboles $R_{10}$, $R_{11}$ et $R_{12}$ représentent chacun, indépendamment les uns des autres, un groupe carboxy, alcoxycarbonyle inférieur, tri-(alkyle inférieur)-silyloxycarbonyle ou un groupe carbamoyle non substitué ou mono- ou di-substitué par des groupes alkyles en $C_1$–$C_{18}$ ou par des groupes phényles ou phénylalkyles inférieurs non substitués ou mono-, di- ou poly-substitués dans la partie phényle par des groupes alkyles inférieurs, hydroxy, alcoxy inférieurs, alcanoyloxy inférieurs, tri-(alkyle inférieur)-silyloxy, alkylène-dioxy inférieurs, des halogènes et/ou des groupes trifluorométhyles, ou un groupe carbamoyle substitué par un groupe alkylène inférieur, $R_{10}$ pouvant également représenter un groupe carbamoyle substitué par un groupe carbamoyl-méthyle ou L-1 ou D-1-carbamoyléthyle et $R_{11}$ pouvant également représenter l'hydrogène, l'expression «inférieur», telle qu'elle est utilisée ci-dessus, désignant un reste qui contient jusqu'à 7 atomes de carbone inclus.

2. Dérivés silylés de glucosamines de formule I selon la revendication 1, dans laquelle:

X représente le groupe carbonyle,

$R_1$, $R_4$ et $R_6$ des groupes tri-(alkyle inférieur)-silyles,

$R_2$ représente un groupe alkyle inférieur non substitué ou substitué par des halogènes, des groupes hydroxy ou tri-(alkyle inférieur)-silyloxy ou un groupe phényle non substitué ou substitué par des groupes alkyles inférieurs, alcoxy inférieurs, des halogènes ou des groupes amino,

$R_3$ repésentent l'hydrogène ou un groupe alkyle en $C_1$–$C_3$,

$R_7$, $R_9$ et $R_{13}$ représentent l'hydrogène,

$R_8$ un groupe alkyle inférieur, hydroxyalkyle inférieur, tri-(alkyle inférieur)-silyloxy-(alkyle inférieur), mercaptoalkyle inférieur ou tri-(alkyle inférieur)-silylmercapto-(alkyle inférieur), $R_7$ et $R_8$ pouvant également former ensemble un groupe triméthylène,

$R_{10}$ et $R_{12}$ représentent chacun, indépendamment l'un de l'autre, un groupe carboxy, alcoxycarbonyle inférieur, tri-(alkyle inférieur)-silyloxycarbonyle, ou carbamoyle non substitué ou mono- ou disubstitué par des groupes alkyles en $C_1$–$C_{18}$ ou par un groupe phényle ou phénylalkyle inférieur non substitué ou mono-, di- ou poly-substitué dans la partie phényle par des groupes alkyles inférieurs, hydroxy, alcoxy inférieurs, alcanoyloxy inférieurs, tri-(alkyle inférieur)-silyloxy, alkylène-dioxy inférieurs, des halogènes et/ou des groupes trifluorométhyles, ou un groupe carbamoyle substitué par un groupe alkylène inférieur, $R_{10}$ pouvant également représenter un groupe carbamoyle substitué par un groupe carbamoylméthyle ou L-1- ou D-1-carbamoyléthyle, et

$R_{11}$ représente l'hydrogène, un groupe tri-(alkyle inférieur)-silyloxy-carbonyle ou un reste carboxy.

3. Dérivés silylés de glucosamines de formule I selon la revendication 1 ou 2 dans laquelle $R_3$ représente l'hydrogène.

4. Dérivés silylés de glucosamines de formule I selon la revendication 1 dans laquelle:

X représente l groupe carbonyle,

$R_1$, $R_4$ et $R_6$ des groupes tri-(alkyle inférieur)-silyles,

$R_2$ un groupe alkyle inférieur ou phényle,

$R_3$ l'hydrogène ou un groupe méthyle,

$R_7$, $R_9$ et $R_{13}$ l'hydrogène,

$R_8$ un groupe alkyle inférieur, hydroxyalkyle inférieur, tri-(alkyle inférieur)-silyloxy-(alkyle inférieur), mercaptométhyle ou tri-(alkyle inférieur)-silylmercapto-méthyle, $R_7$ et $R_8$ pouvant également former ensemble le groupe triméthylène,

$R_{10}$ et $R_{12}$ représentent chacun, indépendamment l'un de l'autre, un groupe carboxy, tri-(alkyle inférieur)-silyloxy-carbonyle ou carbamoyle, et

$R_{11}$ représente l'hydrogène, un groupe tri-(alkyle inférieur)- silyloxycarbonyle ou un groupe carboxy.

5. Dérivés silylés de glucosamines selon l'une quelconque des revendications 1 à 4, dans lesquels

$R_1$, $R_4$ et $R_6$ représentent des groupes triméthylsilyles.

6. Dérivés silylés de glucosamines de formule I selon la revendication 1 dans laquelle:
X représente le groupe carbonyle,
$R_1$, $R_3$ et $R_6$ des groupes triméthylsilyles,
$R_2$ un groupe alkyle inférieur ou phényle,
$R_3$ l'hydrogène ou un groupe méthyle,
$R_7$, $R_9$ et $R_{13}$ l'hydrogène,
$R_8$ un groupe alkyle en $C_1$–$C_3$, hydroxyméthyle, 1-hydroxyéthyle, triméthylsilyloxyméthyle, mercaptométhyle ou triméthylsilylmercaptométhyle,
$R_{10}$ représente un groupe carbamoyle,
$R_{11}$ l'hydrogène et
$R_{12}$ un groupe carboxy, triméthylsilyloxycarbonyle ou carbamoyle.

7. Les composés suivants selon la revendication 1:
le 2-benzoylamino-2-désoxy-3-O-{[L-1-(D-1-carbamoyl-3-triméthylsilyloxycarbonyl-propyl)-carbamoyl-éthyl]-carbamoyl-méthyl}-1,4,6-O-tris-triméthylsilyl-α,β-D-glucose, et
le 2-benzoylamino-2-désoxy-3-O-{[L-1-carbamoyl-3-carboxy-propyl)-carbamoyl-éthyl]-carbamoyl-méthyl}-1,4,6-O-tris-triméthylsilyl-α,β-D-glucose.

8. Les composés suivants selon la revendication 1:
le 2-acétylamino-2-désoxy-3-O-{D-1-[D-1-carbamoyl-3-triméthylsilyloxycarbonyl-propyl]-carbamoyléthyl)-carbamoyl-méthyl}-1,4,6-O-tris-triméthylsilyl-α,β-D-glucose, et
le 2-acétylamino-2-désoxy-3-o-{D-1-(L-1-[D-1-carbamoyl-3-carboxy-propyl]-carbamoyl-éthyl)-carbamoylméthyl}-1,4,6-O-tris-triméthylsilyl-α,β-D-glucose.

9. Les composés suivants selon la revendication 1:
le 2-acétylamino-2-désoxy-3-O-{[L-1-(D-1-carbamoyl-3-triméthylsilyloxycarbonyl-propyl)-carbamoyl-éthyl]-carbamoyl-méthyl}-1,4,6-O-tris-triméthylsilyl-α,β-D-glucose, et
le 2-acétylamino-2-désoxy-3-O-{[L-1-carbamoyl-3-carboxy-propyl)-carbamoyl-éthyl]-carbamoyl-méthyl}-1,4,6-O-tris-triméthylsilyl-α,β-D-glucose.

10. Les composés suivants selon la revendication 1:
le 2-propionylamino-2-désoxy-3-O-{[L-(D-1-carbamoyl-3-triméthylsilyloxycarbonyl-propyl)-carbamoyl-éthyl]-carbamoyl-méthyl}-1,4,6-O-tris-triméthylsilyl-α,β-D-glucose, et
le 2-propionylamino-2-désoxy-3-O-{[L-1-carbamoyl-3-carboxy-propyl)-carbamoyl-éthyl]-carbamoyl-méthyl}-1,4,6-O-tris-triméthylsilyl-α,β-D-glucose.

11. Composés de formule I selon l'une quelconque des revendications 1 à 10, possédant une activité immunomodulante.

12. Composition pharmaceutique contenant un composé selon l'une quelconque des revendications 1 à 10, avec un véhicule acceptable pour l'usage pharmaceutique.

13. Composés de formule I selon l'une quelconque des revendications 1 à 10, pour l'utilisation dans un procédé pour le traitement thérapeutique de l'organisme humain ou animal.

14. Utilisation de l'un des composés mentionnés dans l'une quelconque des revendications 1 à 10, pour la préparation d'un agent immunisant ou d'un vaccin par voie non chimique.

15. Utilisation de l'un des composés mentionnés dans l'une quelconque des revendications 1 à 10, en tant qu'adjuvant en mélange avec des vaccins.

16. Procédé de préparation des composés revendiqués dans l'une quelconque des revendications 1 à 10, caractérisé en ce que l'on fait réagir de manière connue en soi un composé de formule:

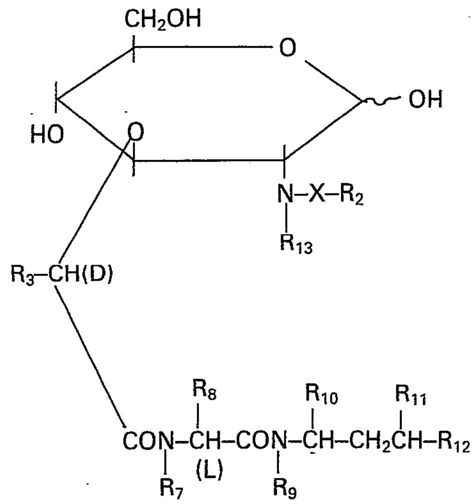

(II)

dans laquelle les substituants sont tels que définis dans l'une quelconque des revendications 1 à 8, avec un agent silylant introduisant un reste tri-(alkyle inférieur)-silyle, et le cas échéant, on scinde l'ester silylique obtenu de formule I selon la revendication 1 par l'eau en éther silylique de formule I.

17. Les composés qu'on peut obtenir par les procédés de la revendication 16.

**Revendications pour l'Etat contractant: AT**

1. Procédé de préparation de dérivés silylés de glycosamines de formule:

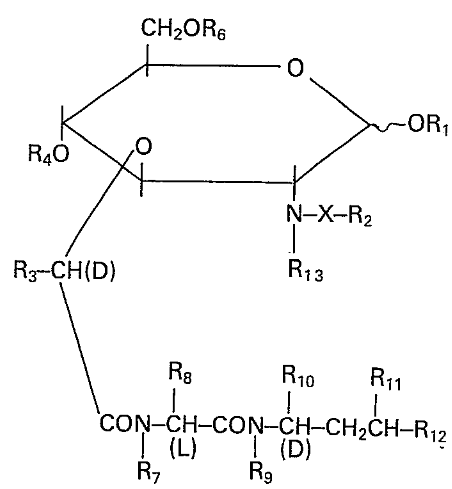

(I)

dans laquelle:

X représente un groupe carbonyle,

$R_1$, $R_4$ et $R_6$ représentent des groupes tri-(alkyle inférieur)-silyles,

$R_2$ représente un groupe alkyle inférieur non substitué ou substitué par des groupes hydroxy, alcoxy inférieurs, tri-(alkyle inférieur)-silyloxy, alcanoyloxy inférieurs, des halogènes, des groupes mercapto, alkylmercapto inférieurs, tri-(alkyle inférieur)-silylmercapto, alcanoylmercapto inférieurs, carboxy, alcoxycarbonyle inférieurs et/ou carbamoyles ou un groupe phényle non substitué ou mono-, di-, ou poly-substitué par des groupes alkyles inférieurs, hydroxy, alcoxy inférieurs, alcanoyloxy inférieurs, tri-(alkyle inférieur)-silyloxy, alkylène inférieur-dioxy, des halogènes, des groupes amino et/ou trifluorométhyle,

$R_3$, $R_7$ et $R_{13}$ représentent l'hydrogène ou des groupes alkyles inférieurs,

$R_8$ représente l'hydrogène, un groupe alkyle inférieur non substitué ou substitué par des groupes hydroxy, alcoxy inférieurs, tri-(alkyle inférieur)-silyloxy, alcanoyloxy inférieurs, des halogènes, des groupes mercapto, alkylmercapto inférieurs, tri-(alkyle inférieur)-silylmercapto ou alcanoylmercapto inférieurs, un groupe cycloalkyle en $C_5$–$C_6$, un groupe cycloalkyl-inférieur dont la partie cycloalkyle contient 5 ou 6 atomes de carbone, un groupe phényle ou phénylalkyle inférieur non substitué ou mono-, di-, ou poly-substitué dans la partie phényle par des groupes alkyles inférieurs, hydroxy, alcoxy inférieurs, alcanoyloxy inférieurs, tri-(alkyle inférieur)-silyloxy, alkylène-dioxy inférieurs, des halogènes et/ou des groupes trifluorométhyles, un groupe hétérocyclyle ou hétérocyclyl-alkyle inférieur contenant chacun 5 ou 6 chaînons cycliques et un ou deux atomes d'azote dans le noyau hétérocyclique éventuellement condensé sur un noyau benzénique,

$R_7$ et $R_8$ peuvent également former un groupe alkylène en $C_3$ ou $C_4$,

$R_9$ représente l'hydrogène ou un groupe alkyle inférieur, et les symboles $R_{10}$, $R_{11}$ et $R_{12}$ représentent chacun, indépendamment les uns des autres, un groupe carboxy, alcoxycarbonyle inférieur, tri-(alkyle inférieur)-silyloxycarbonyle ou un groupe carbamoyle non substitué ou mono- ou di-substitué par des groupes alkyles en $C_1$–$C_{18}$ ou par des groupes phényles ou phénylalkyles inférieurs non substitués ou mono- di- ou poly-substitués dans la partie phényle par des groupes alkyles inférieurs, hydroxy, alcoxy inférieurs, alcanoyloxy inférieurs, tri-(alkyle inférieur)-silyloxy, alkylène-dioxy inférieurs, des halogènes et/ou des groupes trifluorométhyles, ou un groupe carbamoyle substitué par un groupe alkylène inférieur, $R_{10}$ pouvant également représenter un groupe carbamoyle substitué par un groupe carbamoyl-méthyle ou L-1- ou D-1-carbamoyléthyle et $R_{11}$ pouvant également représenter l'hydrogène, l'expression «inférieur», telle qu'elle est utilisée ci-dessus, désignant un reste qui contient jusqu'à 7 atomes de carbone inclus,

caractérisé en ce que l'on fait réagir de manière connue en soi un composé de formule II:

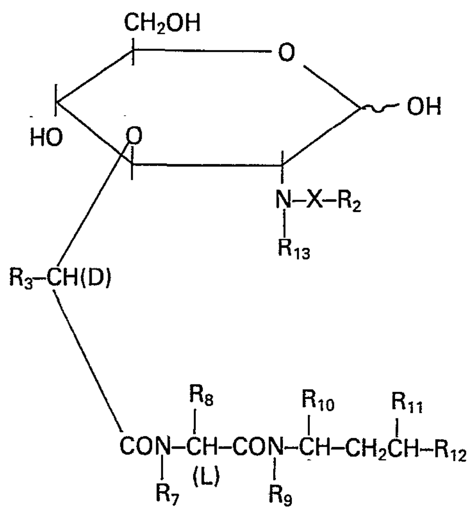

(II)

dans laquelle des substituants sont tels que définis ci-dessus, avec un agent silylant introduisant un groupe tri-(alkyle inférieur)-silyle et le cas échéant on convertit l'ester silylique obtenu de formule I en éther silylique de formule I par l'eau.

2. Procédé selon la revendication 1, caractérisé en ce que l'on prépare des dérivés silylés de glucosamines de formule I selon la revendication 1, dans laquelle:

X représente le groupe carbonyle,

$R_1$, $R_4$ et $R_6$ des groupes tri-(alkyle inférieur)-silyles,

$R_2$ représente un groupe alkyle inférieur non substitué ou substitué par des halogènes, des groupes hydroxy ou tri-(alkyle inférieur)-silyloxy ou un groupe phényle non substitué ou substitué par des groupes alkyles inférieurs, alcoxy inférieurs, des halogènes ou des groupes amino,

$R_3$ représente l'hydrogène ou un groupe alkyle en $C_1$–$C_3$,

$R_7$, $R_9$ et $R_{13}$ représentent l'hydrogène,

$R_8$ un groupe alkyle inférieur, hydroxyalkyle inférieur, tri-(alkyle inférieur)-silyloxy-(alkyle inférieur), mercaptoalkyle inférieur ou tri-(alkyle inférieur)-silylmercapto-(alkyle inférieur), $R_7$ et $R_8$ pouvant également former ensemble un groupe triméthylène,

$R_{10}$ et $R_{12}$ représentent chacun, indépendamment l'un de l'autre, un groupe carboxy, alcoxycarbonyle inférieur, tri-(alkyle inférieur)-silyloxycarbonyle, ou carbamoyle non substitué ou mono- ou disubstitué par des groupes alkyles en $C_1$–$C_{18}$ ou par un groupe phényle ou phénylalkyle inférieur non substitué ou mono-di-, ou poly-substitué dans la partie phényle par des groupes alkyles inférieurs, hydroxy, alcoxy inférieurs, alcanoyloxy inférieurs, tri-(alkyle inférieur)-silyloxy, alkylène-dioxy inférieurs, des halogènes et/ou des groupes trifluorométhyles, ou un groupe carbamoyle substitué par un groupe alkylène inférieur, $R_{10}$ pouvant également représenter un groupe carbamoyle substitué par un groupe carbamoylméthyle ou L-1- ou D-1-carbamoyléthyle et $R_{11}$ représente l'hydrogène, un groupe tri-(alkyle inférieur)-silyloxy-carbonyle ou un reste carboxy.

3. Procédé selon la revendication 1 ou 2, caractérisé en ce que l'on prépare des dérivés silyles de glucosamines pour lesquels, dans la formule I indiquée, $R_3$ représente l'hydrogène.

4. Procédé selon l'une quelconque des revendications 1 à 3, caractérisé en ce que l'on prépare des dérivés silylés de glucosamines de formule I selon la revendication 1, dans laquelle:

X représente le groupe carbonyle,
$R_1$, $R_4$ et $R_6$ des groupes tri-(alkyle inférieur)-silyles,
$R_2$ un groupe alkyle inférieur ou phényle,
$R_3$ l'hydrogène ou un groupe méthyle,
$R_7$, $R_9$ et $R_{13}$ l'hydrogène,
$R_8$ un groupe alkyle inférieur, hydroxyalkyle inférieur, tri-(alkyle inférieur)-silyloxy-(alkyle inférieur), mercaptométhyle ou tri-(alkyle inférieur)-silylmercapto-méthyle, $R_7$ et $R_8$ pouvant également former ensemble le groupe triméthylène,
$R_{10}$ et $R_{12}$ représentent chacun, indépendamment l'un de l'autre, un groupe carboxy, tri-(alkyle inférieur)-silyloxy-carbonyle ou carbamoyle, et
$R_{11}$ représente l'hydrogène, un groupe tri-(alkyle inférieur)-silyloxycarbonyle ou un groupe carboxy.

5. Procédé selon l'une quelconque des revendications 1 à 4, caractérisé en ce que l'on prépare des composés de formule I selon la revendication 1, dans laquelle:

$R_1$, $R_4$ et $R_6$ représentent des groupes triméthylsilyles.

6. Procédé selon l'une quelconque des revendications 1 à 4, caractérisé en ce que l'on prépare des dérivés silylés de glycosamines de formule I selon la revendication 1 dans laquelle:

X représente le groupe carbonyle,
$R_1$, $R_4$ et $R_6$ des groupes triméthylsilyles,
$R_2$ un groupe alkyle inférieur ou phényle,
$R_3$ l'hydrogène ou un groupe méthyle,
$R_7$, $R_9$ et $R_{13}$ l'hydrogène,
$R_8$ un groupe alkyle en $C_1$–$C_3$, hydroxyméthyle, 1-hydroxyéthyle, triméthylsilyloxyméthyle, mercaptométhyle ou triméthylsilylmercapto-méthyle,
$R_{10}$ représente un groupe carbamoyle,
$R_{11}$ l'hydrogène, et
$R_{12}$ un groupe carboxy, triméthylsilyloxycarbonyle ou carbamoyle.

7. Procédé selon la revendication 1, caractérisé en ce que l'on prépare:
le 2-benzoylamino-2-désoxy-3-O-{[L-1-(D-1-carbamoyl-3-triméthylsilyloxycarbonyl-propyl)-carbamoyl-éthyl]-carbamoylméthyl}-1,4,6-O-tris-triméthylsilyl-α,β-D-glucose, ou
le 2-benzoylamino-2-désoxy-3-O-{[L-1-(D-1-carbamoyl-3-carboxy-propyl)-carbamoyl-éthyl]-carbamoyl-méthyl}-1,4,6-O-tris-triméthylsilyl-α,β-D-glucose.

8. Procédé selon la revendication 1, caractérisé en ce que l'on prépare:
le 2-acétylamino-2-désoxy-3-O-{D-1-[D-1-carbamoyl-3-triméthylsilyloxycarbonyl-propyl]-carbamoyl-éthyl)-carbamoyl-méthyl}-1,4,6-O-tris-triméthylsilyl-α,β-D-glucose, ou
le 2-acétylamino-2-désoxy-3-O-{D-1-(L-1-[-D-1-carbamoyl-3-carboxy-propyl]-carbamoyl-éthyl)-carbamoyl-méthyl}-1,4,6-O-tris-triméthylsilyl-α,β-D-glucose.

9. Procédé selon la revendication 1, caractérisé en ce que l'on prépare:
le 2-acétylamino-2-désoxy-3-O-{[L-1-(D-1-carbamoyl-3-triméthylsilyloxycarbonyl-propyl)-carbamoyl-éthyl]-carbamoyl-méthyl}-1,4,6-O-tris-triméthylsilyl-α,β-D-glucose, ou
le 2-acétylamino-2-désoxy-3-O-{[L-1-carbamoyl-3-carboxy-propyl)-carbamoyl-éthyl]-carbamoyl-méthyl}-1,4,6-O-tris-triméthylsilyl-α,β-D-glucose.

10. Procédé selon la revendication 1, caractérisé en ce que l'on prépare:
le 2-propionylamino-2-désoxy-3-O-{[L-(D-1-carbamoyl-3-triméthylsilyloxycarbonyl-propyl)-carbamoyl-éthyl]-carbamoyl-méthyl}-1,4,6-O-tris-triméthylsilyl-α,β-D-glucose, ou
le 2-propionylamino-2-désoxy-3-O-{[L-1-carbamoyl-3-carboxy-propyl)-carbamoyl-éthyl]-carbamoyl-méthyl}-1,4,6-O-tris-triméthylsilyl-α,β-D-glucose.

11. Procédé selon l'une quelconque des revendications 1 à 10, caractérisé en ce qu'on utilise en tant qu'agents silylants des halogénures de tri-(alkyle inférieur)-silyle, des hexa-(alkyle inférieur)-disilazanes, des tri-(alkyle inférieur)-silylamines, des tri-(alkyle inférieur)-silyldiéthylamines, des tri-(alkyle inférieur)-silylacétamides, des bis-[tri-(alkyle inférieur)-silyl]-acétamides ou des tri-(alkyle inférieur)-silylsulfonamides.

12. Procédé de préparation d'une composition pharmaceutique, caractérisé en ce que l'on mélange un composé de formule I selon la revendication 1 avec un véhicule acceptable pour l'usage pharmaceutique.